# EUROPEAN PATENT SPECIFICATION

(11) **EP 3 585 443 B1**
(45) Date of publication and mention of the grant of the patent: **07.05.2025**
(21) Application number: 18758507.0
(22) Date of filing: 21.02.2018
(51) Int. Cl.: C12N 15/63, C12N 15/67, C12N 15/85

(54) **REGULATION OF GENE EXPRESSION BY APTAMER-MEDIATED ACCESSIBILITY OF POLYADENYLATION SIGNALS**
REGULIERUNG DER GENEXPRESSION DURCH APTAMERVERMITTELTE ZUGÄNGLICHKEIT VON POLYADENYLIERUNGSSIGNALEN
RÉGULATION D'EXPRESSION GÉNIQUE PAR MODULATION MÉDIÉE PAR APTAMÈRE DE L'ACCESSIBILITÉ DE SIGNAUX DE POLYADÉNYLATION

(30) Priority: 21.02.2017 US 201762461689 P
(43) Date of publication of application: 01.01.2020
(73) Proprietor: Meiragtx UK II Limited, London N1 7NQ (GB)
(72) Inventor: GUO, Xuecui, Oyster Bay, New York 11771 (US); HAN, Joonhee, Lawrence Township, NJ 08648 (US); ZHONG, Zhaojing, Bronx, NY 10461 (US)
(74) Representative: Pohlman, Sandra M.
(86) International application number: PCT/US2018/019056
(87) International publication number: WO 2018/156658

(56) References cited:
- WO-A1-2010/141085
- WO-A1-2016/126747
- WO-A1-2016/126747
- WO-A1-2016/205764
- WO-A1-2016/205764
- US-A1- 2010 221 821
- CHRISTIAN BERENS ET AL: "RNA aptamers as genetic control devices: The potential of riboswitches as synthetic elements for regulating gene expression", BIOTECHNOLOGY JOURNAL, vol. 10, no. 2, 1 February 2015 (2015-02-01), DE, pages 246 - 257, XP055279945, ISSN: 1860-6768, DOI: 10.1002/biot.201300498
- MAIKE SPÖRING ET AL: "Aptamer-Mediated Control of Polyadenylation for Gene Expression Regulation in Mammalian Cells", ACS SYNTHETIC BIOLOGY, 27 October 2020 (2020-10-27), Washington, DC,USA, XP055748643, ISSN: 2161-5063, DOI: 10.1021/acssynbio.0c00222

## Description

### FIELD OF THE INVENTION

The invention provides polynucleotide constructs for the regulation of gene expression by aptamer-based modulation of the accessibility of one or more polyadenylation signals and methods of using the polynucleotide constructs to regulate gene expression in response to the presence or absence of a ligand that binds the aptamer. The polynucleotide construct contains a riboswitch comprising an aptamer and an effector stem loop, wherein the effector stem loop comprises a polyadenylation signal sequence.

### BACKGROUND OF THE INVENTION

Messenger RNAs (mRNAs) in eukaryotic cells are produced from pre-mRNA transcripts by extensive post-transcriptional processing, including 5' end capping, removal of introns by splicing, and 3' end cleavage and polyadenylation. The 3' end of almost all eukaryotic mRNAs comprises a poly(A) tail-a homopolymer of 20 to 250 adenosine residues. The poly(A) tail is added to pre-mRNA in the nucleus by cleavage and polyadenylation, a process catalyzed by a large complex of proteins. Addition of a poly(A) tail depends on the presence of multiple elements including the highly conserved AATAAA (or its variant ATTAAA) polyadenylation signal sequence found upstream of the polyadenylation site, and other upstream elements ("USE"), as well as a T or GT-rich downstream element ("DSE"). Addition of a poly(A) tail to mRNA protects the message from degradation, among other functions.

WO 2010/141085 A1 (Myung J. et al.) teaches a method for inducing riboswitch-mediated transcription termination using a promoter operably linked to a riboswitch aptamer. Riboswitches that regulate splicing of RNA are described in US 2010/221821 A1 (Breaker, R. et al.)*.* A review article by Berens, C. et al. (Biotechnology Journal, 10(2), 246-257) discusses riboswitches in the regulation of gene expression. WO 2016/126747 A1 (Boyne, A. et al.) discloses the use of riboswitches to regulate gene expression by controlling alternative splicing. WO 2016/205764 A1 (Severinov, K. et al.) relates to a method of targeted regulation of gene expression using an RNA-targeting CRISPR effector protein and at least one targeting nucleic acid component such as a guide RNA (gRNA).

### SUMMARY OF THE INVENTION

The invention is set out in the appended set of claims. In one aspect, the present invention provides a polynucleotide cassette for the regulation of the expression of a target gene comprising a riboswitch wherein the riboswitch comprises an effector stem-loop and an aptamer, wherein the effector stem-loop comprises a polyadenylation signal, and wherein the aptamer and effector stem-loop are linked by an alternatively shared stem arm comprising sequence that is complementary to the unshared arm of the aptamer stem and to the unshared arm of the effector stem loop. In one embodiment, the aptamer binds a small molecule ligand.

In embodiments, the portion of the alternatively shared stem arm that is complementary to sequence in the aptamer stem and to sequence in the effector stem loop is 4 to 8 nucleotides, 5 to 7 nucleotides, 5 nucleotides, or 6 nucleotides. In embodiments, the aptamer stem is 6 to 12 base pairs, 7 to 10 base pairs, 8 base pairs, or 9 base pairs. In embodiments, the stem of the effector stem loop is 4 to 24 base pairs, 5 to 20 base pairs, 9 to 14 base pairs, 9 base pairs, 10 base pairs, 11 base pairs or 12 base pairs.

In one embodiment, the effector stem-loop is positioned 3' of the aptamer such that the alternatively shared stem arm comprises all or a portion of the 3' aptamer stem arm and all or a portion of the 5' arm of the effector stem. In one embodiment, the effector stem-loop is positioned 5' of the aptamer such that the alternatively shared stem arm comprises all or a portion of the 5' aptamer stem arm and all or a portion of the 3' arm of the effector stem. In one embodiment, the polyadenylation signal is AATAAA or ATTAAA. In one embodiment, the polyadenylation signal is a downstream element (DSE). In one embodiment, the polyadenylation signal is an upstream sequence element (USE).

In one embodiment, the polynucleotide cassette comprises two riboswitches of the present invention, wherein the effector stem loop of the first riboswitch comprises all or part of the polyadenylation signal AATAAA or ATTAA and the effector stem loop of the second riboswitch comprises all or part of the downstream element (DSE). In one embodiment, the two riboswitches each comprise an aptamer that binds the same ligand. In one embodiment, the two riboswitches comprise different aptamers that bind different ligands.

In one aspect, the present invention provides a method of modulating the expression of a target gene comprising
(a) inserting one or more of the polynucleotide cassettes of the present invention into the 3' untranslated region of a target gene,
(b) introducing the target gene comprising the polynucleotide cassette into a cell *in vitro* or *ex vivo,* and
(c) exposing the cell to a ligand that binds the aptamer in an amount effective to increase expression of the target gene.

In one embodiment, the ligand is a small molecule. In one embodiment, the effector stem loop is positioned 3' of the aptamer such that the alternatively shared stem arm comprises all or a portion of the 3' aptamer stem arm and all or a portion of the 5' arm of the effector stem. In one embodiment, the polyadenylation signal is AATAAA or ATTAAA. In one embodiment, the effector stem loop is positioned 5' of the aptamer such that the alternatively shared stem arm comprises all or a portion of the 5' aptamer stem arm and all or a portion of the 3' arm of the effector stem. In one embodiment, the polyadenylation signal is a downstream element (DSE). In one embodiment, two riboswitches are inserted into the 3' untranslated region ("UTR") of the target gene, wherein the effector stem loop of the first riboswitch comprises all or part of the polyadenylation signal AATAAA or ATTAA and the effector stem loop of the second riboswitch comprises all or part of the downstream element (DSE). In one embodiment, the two riboswitches each comprise an aptamer that binds the same ligand. In one embodiment, the two riboswitches comprise different aptamers that bind different ligands. In one embodiment, the two or more polynucleotide cassettes comprise the same aptamer.

In one embodiment, the target gene comprising the polynucleotide cassette is incorporated in a vector for the expression of the target gene. In one embodiment, the target gene further comprises a gene regulation cassette that modulates target gene expression by aptamer- mediated regulation of alternative splicing. In one embodiment, the vector is a viral vector. In one embodiment, the viral vector is selected from the group consisting of adenoviral vector, adeno-associated virus vector, and lentiviral vector.

In one aspect, the present invention provides a vector comprising a target gene that contains a polynucleotide cassette described herein. In one embodiment, the vector is a viral vector. In one embodiment, the viral vector is selected from the group consisting of adenoviral vector, adeno-associated virus vector, and lentiviral vector. In one embodiment, the target gene further comprises a gene regulation cassette that modulates target gene expression by aptamer-mediated regulation of alternative splicing.

In a further aspect, the invention relates to the polynucleotide cassette or the vector described herein for use in the treatment of cancer, immune disorders, metabolic diseases, rare diseases such as PNH, ocular angiogenesis, dry AMD, type I diabetes, type II diabetes, metabolic syndrome, growth disorders or growth hormone-deficient, anemia due to chronic kidney disease, anemia due to myelodysplasia, anemia due to cancer chemotherapy, cystic fibrosis, hemophilia, muscular dystrophy, thalassemia, sickle cell anemia, hemophilia A or hemophilia B. Another aspect of the invention pertains to the polynucleotide cassette or the vector described herein for use in the modulation of the expression of the target gene. In yet another aspect, the invention provides the polynucleotide cassette or the vector described herein for use as a medicament.

In one aspect, the polynucleotide cassette of the present invention is used in combination with other mechanisms for the regulation of expression of the target gene. In one embodiment, a polynucleotide cassette of the present invention is used in combination with a gene regulation cassette that modulates target gene expression by aptamer-mediated regulation of alternative splicing as described in WO 2016/126747 (PCT/US2016/016234). In other embodiments, the polynucleotide cassette of the present invention used in combination with a gene regulation cassette that modulates target gene expression by aptamer-mediated regulation of self-cleaving ribozymes as described in PCT/US2017/016303. In other embodiments, the polynucleotide cassette of the present invention used in combination with a gene regulation cassette that modulates target gene expression by aptamer-mediated modulation of polyadenylation as described in PCT/US1207/016279.

### BRIEF DESCRIPTION OF THE FIGURES

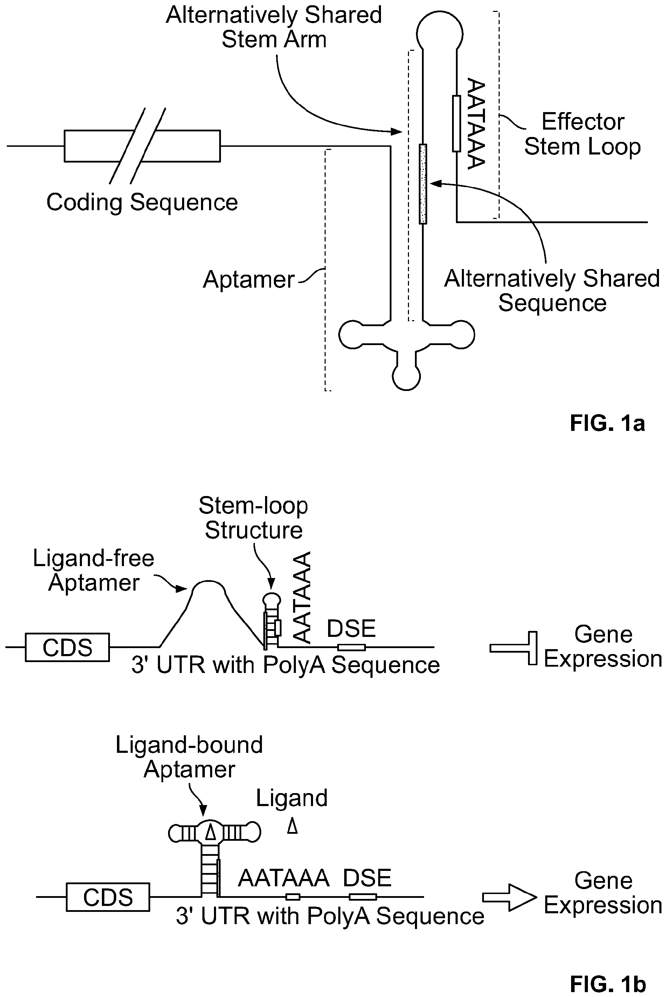

Fig. 1a. A schematic for one embodiment of the invention in which the 3' stem arm of an aptamer is linked to the 5' stem arm of the effector stem-loop via an alternatively shared stem arm (i.e., a stem arm that can form a stem structure with either the aptamer stem or the effector stem, but not both at the same time), and a polyadenylation signal sequence (in this case AATAAA) is located in the stem of the effector stem-loop.
Fig. 1b. Schematics for one embodiment of the invention in which the accessibility of a polyadenylation signal (in this case AATAAA) is regulated by the presence or absence of aptamer ligand. An aptamer linked to an effector stem-loop (with AATAAA sequence embedded in the stem) is inserted in the 3' UTR. The complementary sequence of the 5' arm of the stem-loop structure followed by aptamer sequence is linked to the 5' arm of the stem-loop structure. In the absence of aptamer ligand (top panel), AATAAA sequence is blocked by stem-loop structure formed by the effector stem-loop, inhibiting polyadenylation and thereby suppressing gene expression. In the presence of aptamer ligand, aptamer/ligand binding facilitates the formation of the aptamer P1 stem, thereby disrupting the effector stem-loop structure and leading to the release of AATAAA from being sequestered. The sequence that is shared between the stem of stem-loop structure and aptamer P1 stem is indicated as a thick line.

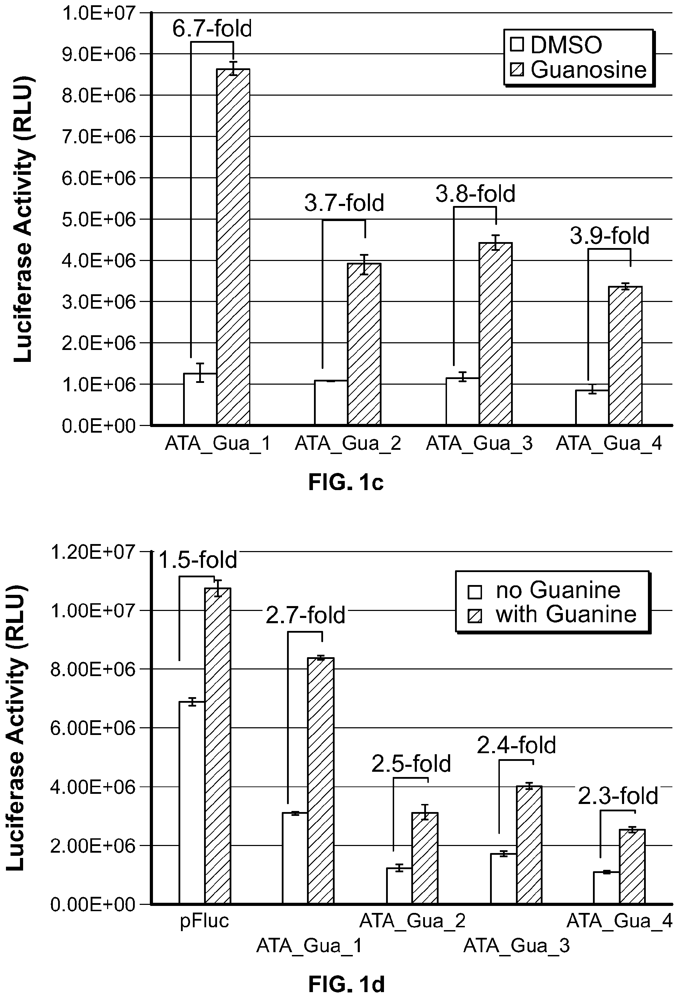

Fig. 1c and Fig. 1d. Regulation of luciferase expression via aptamer-mediated modulation of AATAAA accessibility. HEK 293 cells were transfected with the indicated constructs and treated with DMSO (Fig. 1c) or NaOH (Fig. 1d) as solvent control or 500 µM guanosine (Fig. 1c) or guanine (Fig. 1d). Luciferase activity was expressed as mean±S.D. (n=3), and the induction fold was expressed as the quotient of luciferase activity obtained in the presence of guanosine or guanine divided by the value obtained in the absence of guanosine or guanine.

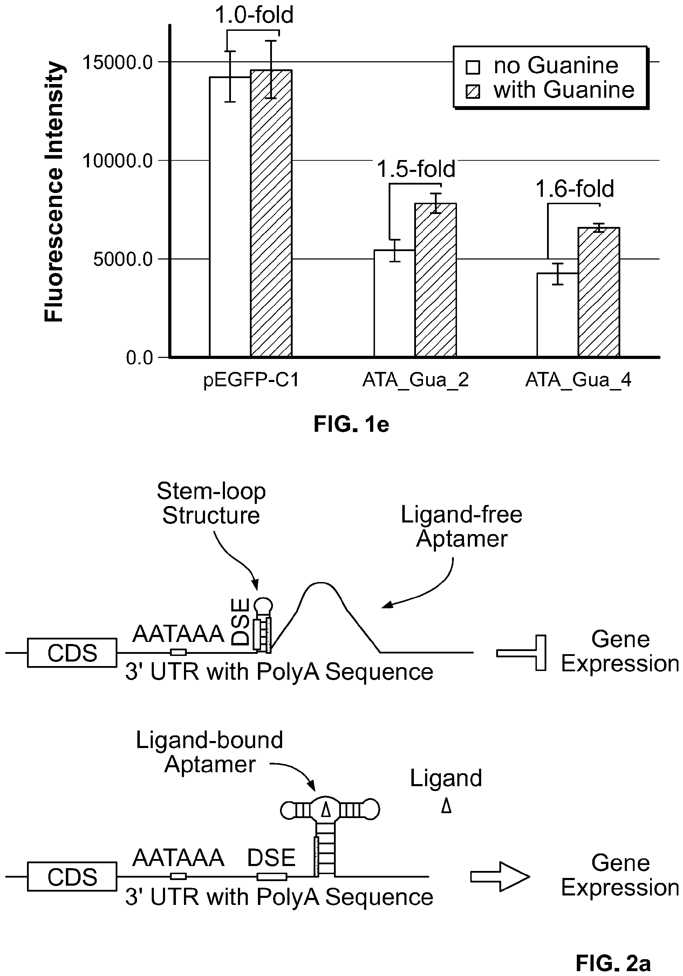

Fig. 1e. Regulation of EGFP expression via aptamer-mediated modulation of accessibility of AATAAA. HEK 293 cells were transfected with the indicated constructs and treated with either NaOH as solvent control or 500 µM guanine. The GFP fluorescence intensity was expressed as mean±S.D. (n=3), and the induction fold was expressed as the quotient of fluorescence intensity obtained in the presence of guanine divided by the value obtained in the absence of guanine.
Fig. 2a. Schematics of modulating the accessibility of DSE in a stem-loop by aptamer/ligand binding. A stem-loop forming structure with DSE sequence embedded in the stem is inserted in the 3' UTR. Aptamer sequence is linked to the 3' arm of the stem-loop forming structure followed by the complementary sequence of the 3' arm of stem-loop structure. In the absence of aptamer ligand (top panel), the DSE sequence is blocked by formation of stem-loop structure, thereby inhibiting polyadenylation and suppressing target gene expression. In the presence of aptamer ligand (bottom panel), aptamer/ligand binding facilitates the formation of the aptamer P1 stem, thereby disrupting the stem-loop structure and leading to release of the DSE sequence from being sequestered. The sequence that is shared between the stem of effector stem-loop structure and aptamer P1 stem is indicated as thick line.

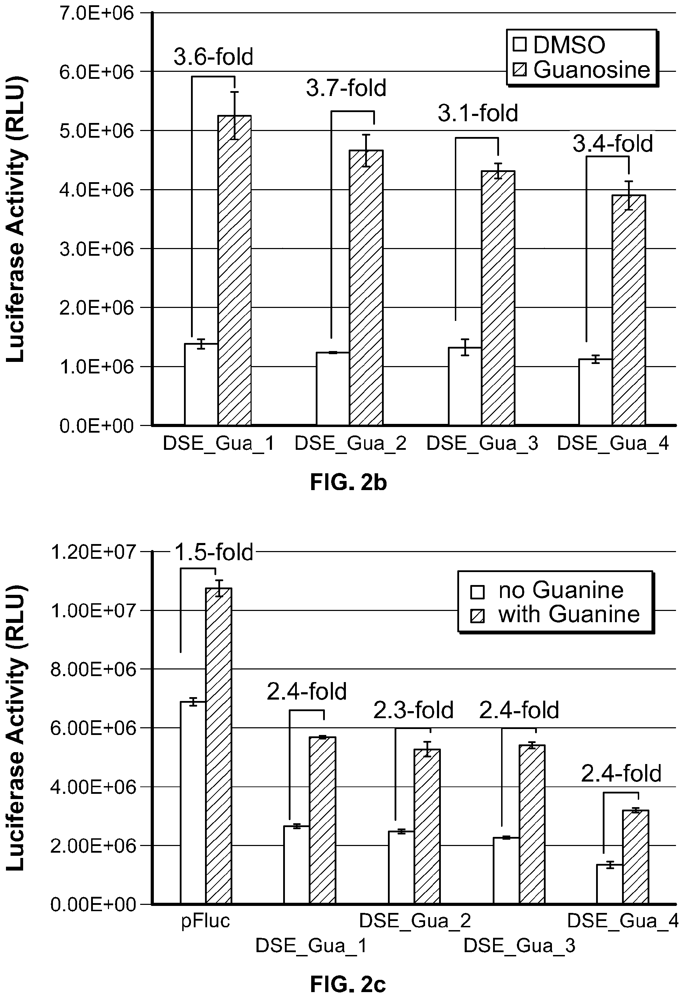

Fig. 2b and Fig. 2c. Regulation of luciferase expression via aptamer-mediated modulation of DSE accessibility. HEK 293 cells were transfected with the indicated constructs and treated with DMSO (Fig. 2b) or NaOH (Fig. 2c) as solvent control or 500 µM guanosine (Fig. 2b) or guanine (Fig. 2c). Luciferase activity is presented as mean±S.D. (n=3), and the induction fold was expressed as the quotient of luciferase activity obtained in the presence of guanosine or guanine divided by the value obtained in the absence of guanosine or guanine.

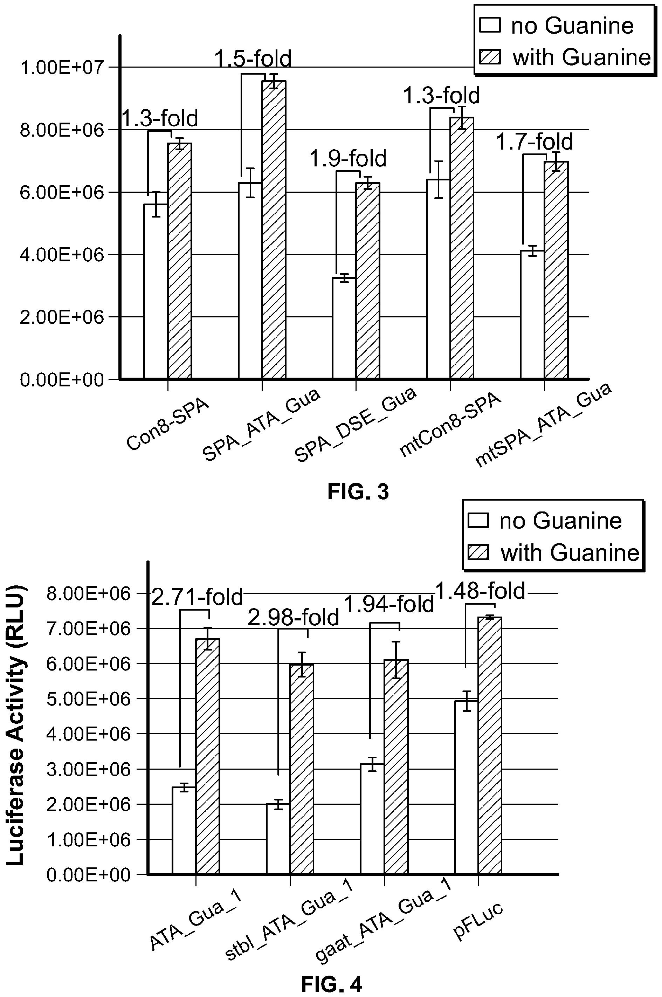

Fig. 3. Regulation of luciferase expression via aptamer-mediated modulation of access to a synthetic polyA sequence.
Fig. 4. Loop sequence in the stem-loop structure affects the polyA-based riboswitch activity. stbl_ATA_Gua_1 has a more stable TTCG loop, ATA_Gua_1 has GAAA loop and gaat_ATA_Gua_1 has GAAT loop.

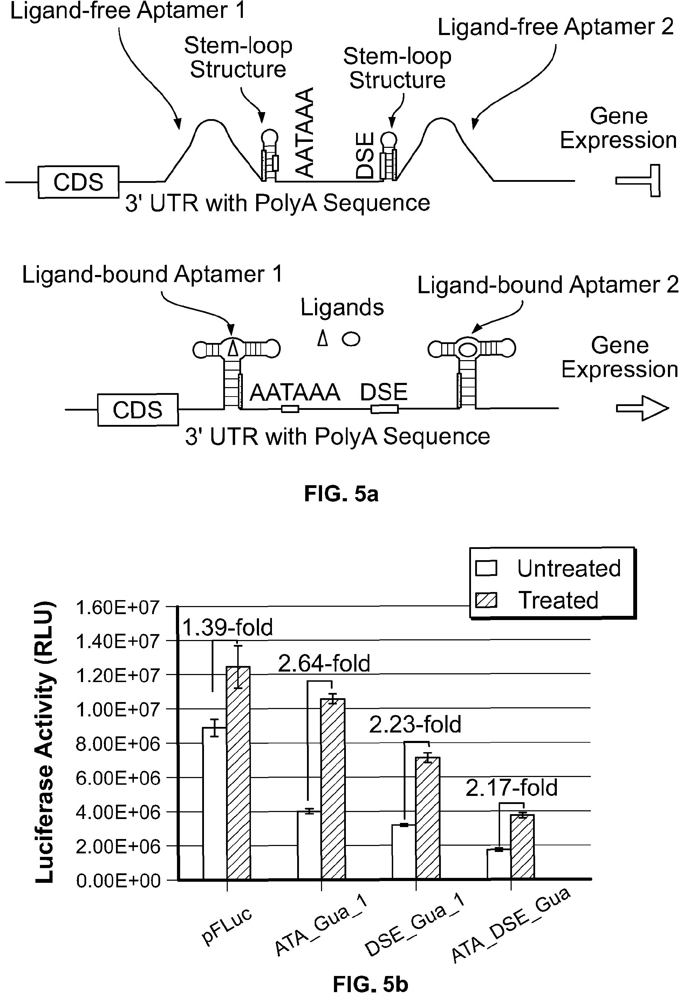

Fig. 5a. Schematics of modulating the accessibility of both AATAAA and DSE sequence simultaneously via aptamer. Two stem-loop structures each embedding AATAAA or DSE sequence are inserted in the 3' UTR of a target gene, and aptamers are linked to each stem-loop structure as described for Fig. 1b and Fig. 2a. Aptamer 1 and aptamer 2 represent the same aptamer or different aptamers that bind the same or different ligands. In the absence of aptamer ligand (top panel), both AATAAA and DSE are sequestered in the stem-loop structures, therefore gene expression is suppressed. In the presence of aptamer ligand (lower panel), when both aptamers bind their ligands, both AATAAA and DSE sequence are released from the stem-loop structure, allowing the target gene to be expressed.
Fig. 5b. HEK 293 cells were transfected with the indicated constructs and treated with NaOH as solvent control or 500 µM guanine. Luciferase activity was expressed as mean±S.D. (n=3), and the induction fold was expressed as the quotient of luciferase activity obtained in the presence of guanine divided by the value obtained in the absence of guanine. Simultaneously sequestering both AATAAA and DSE sequence element in polyA sequence further decreases the basal level of luciferase expression in ATA_DSE_Gua construct.

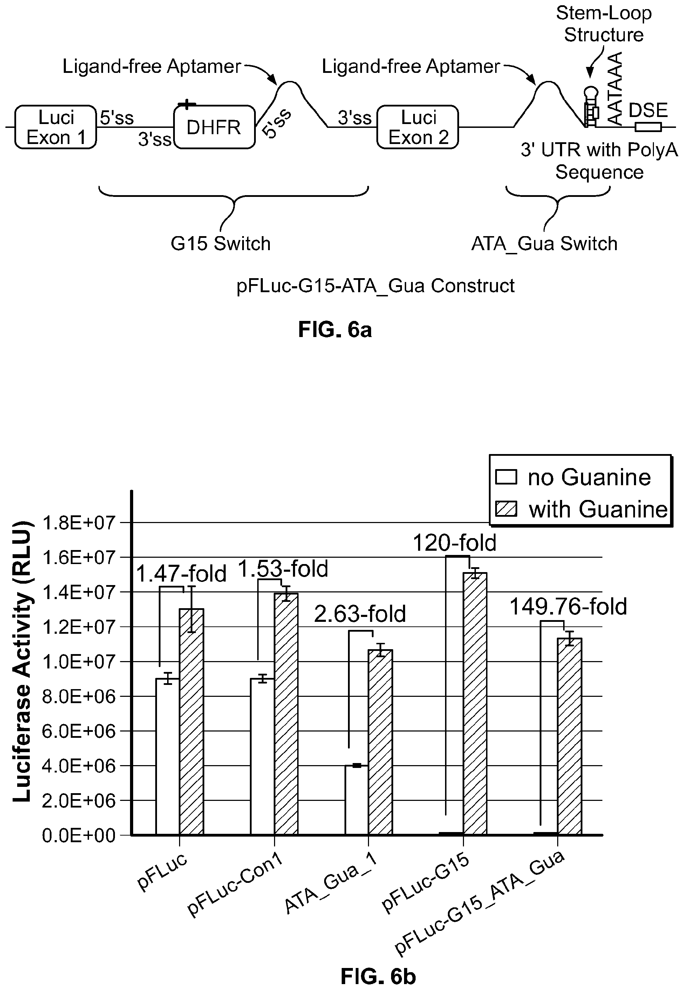

Fig. 6a. Schematics of the dual switch construct in which ATA_Gua riboswitch is in the 3'UTR and the G15 riboswitch is inserted in the luciferase coding sequence.
Fig. 6b. Luciferase activity from HEK 293 cells transfected with the indicated constructs and treated with or without 500 uM guanine. The construct with two switches pFLuc-G15_ATA _Gua generated higher fold induction than the construct with single riboswitch.

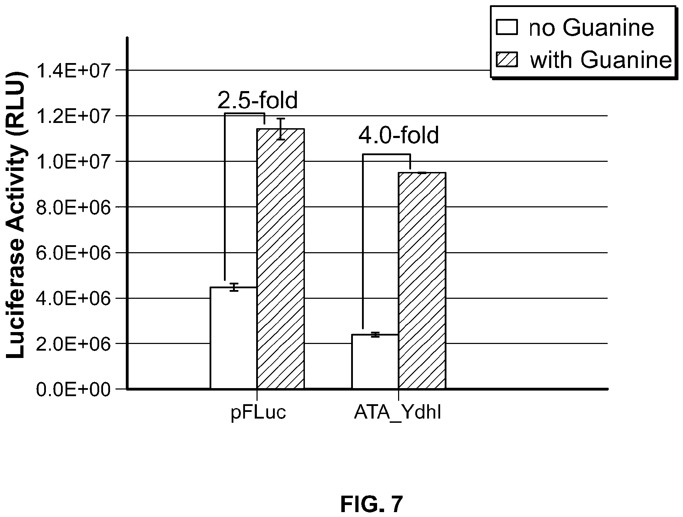

Fig. 7. Regulation of luciferase expression via adenine aptamer-mediated modulation of accessibility of AATAAA. HEK 293 cells were transfected with the indicated constructs and treated with NaOH as solvent control or 1 mM adenine. Luciferase activity was expressed as mean±S.D. (n=3), and the induction fold was expressed as the quotient of luciferase activity obtained in the presence of adenine divided by the value obtained in the absence of adenine.

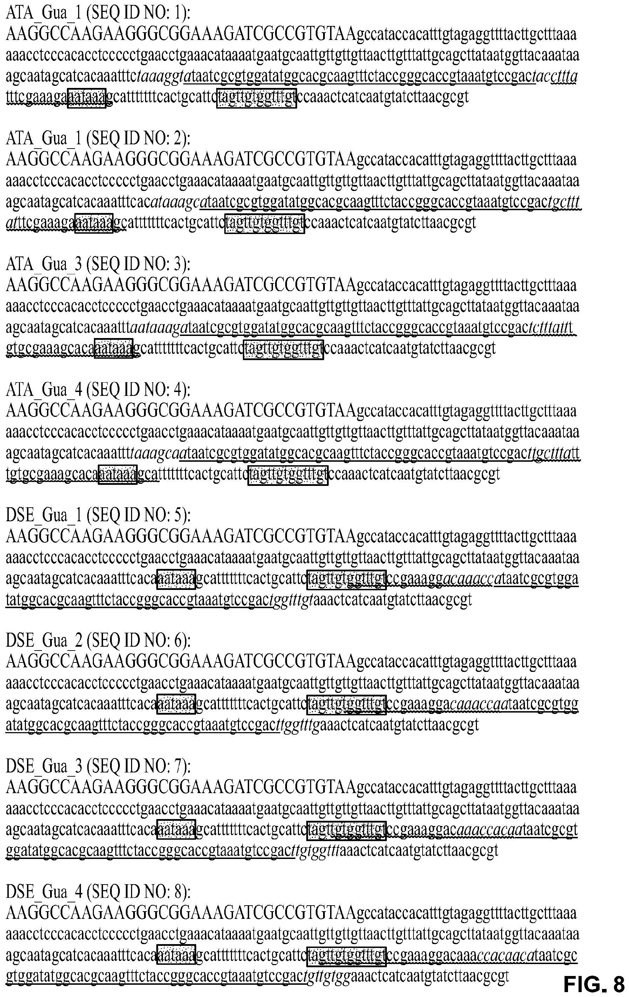

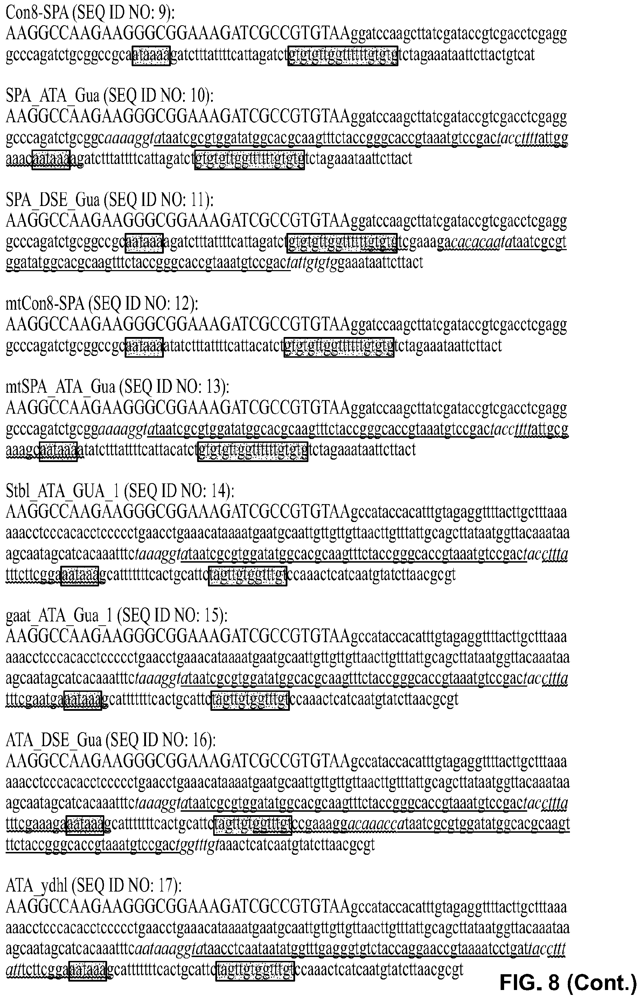

Fig. 8. The 3' UTR of the constructs utilized in the examples. The coding sequence for luciferase gene is in uppercase letters; AATAAA and DSE are highlighted in gray; aptamer sequence is underlined; the stem-loop sequence is wave underlined; and the P1 aptamer stem sequence is in italicized letters.

### DETAILED DESCRIPTION OF THE INVENTION

Regulation of the expression of a target gene (e.g., a therapeutic transgene) is useful or necessary in a variety of situations. In the context of the therapeutic expression of genes, techniques that enable regulated expression of transgenes have the potential to enhance safety by regulating the level of expression and its timing. A regulated system to control protein expression has practical and, in some cases, essential roles for safe and effective therapeutic applications. The invention provides polynucleotide constructs for the regulation of gene expression by aptamer-based modulation of polyadenylation by sequestering one or more polyadenylation signals in a stem-loop structure (the effector stem-loop) that is linked to the aptamer and methods of using the constructs to regulate gene expression in response to the presence or absence of a ligand that binds the aptamer.

The polynucleotide construct contains at least one riboswitch that comprises an effector stem-loop and an aptamer where the effector stem-loop comprises a polyadenylation signal sequence. The aptamer and the effector stem-loop are linked by a shared stem arm that can alternatively form a stem with either the aptamer stem or the effector stem depending on the presence of an aptamer ligand. When an aptamer ligand is present and bound to the aptamer, the aptamer stem (the aptamer P1 stem) is stabilized and forms a stem with the alternatively shared stem arm. Thus, in the presence of ligand, the stem-loop structure formed by the effector step-loop is disfavored and the polyadenylation signal is accessible, allowing polyadenylation to occur leading to enhanced target gene expression. In the absence of ligand, the effector stem-loop forms a stem structure with the alternatively shared stem arm, thereby sequestering the polyadenylation signal sequence, preventing polyadenylation and decreasing target gene expression.

In one embodiment, the effector stem loop is positioned 3' of the aptamer such that the alternatively shared stem arm comprises all or a portion of the 3' aptamer stem arm and all or a portion of the 5' arm of the effector stem loop *(see, e.g.,* Figs. 1a and 1b). In one embodiment, the effector stem loop is positioned 5' of the aptamer such that the alternatively shared stem arm comprises all or a portion of the 5' aptamer stem arm and all or a portion of the 3' arm of the effector stem loop *(see, e.g.,* Fig. 3).

The gene regulation polynucleotide cassette refers to a recombinant DNA construct that, when incorporated into the DNA of a target gene in the 3' UTR, provides the ability to regulate expression of the target gene by aptamer/ligand mediated regulation of polyadenylation. As used herein, a polynucleotide cassette or construct is a nucleic acid (e.g., DNA or RNA) comprising elements derived from different sources (e.g., different organisms, different genes from the same organism, and the like).

### Riboswitch

The polynucleotide cassette comprises a riboswitch. The term "riboswitch" as used herein refers to a regulatory segment of a RNA polynucleotide (or the DNA encoding the riboswitch). A riboswitch in the context of the present invention contains a sensor region (i.e., an aptamer) and an effector step-loop that together are responsible for sensing the presence of a ligand (e.g., a small molecule) and modulating the accessibility of a polyadenylation sequence located in the effector stem-loop. In one embodiment, the riboswitch is recombinant, utilizing polynucleotides from two or more sources. The term "synthetic" as used herein in the context of a riboswitch refers to a riboswitch that is not naturally occurring.

### Effector stem-loop

The effector stem-loop of the riboswitch comprises RNA sequence (or DNA that encodes the RNA sequence) that, in the absence of a ligand binding the sensor region (i.e., an aptamer), forms a stem structure (i.e., a double-stranded region) that reduces the accessibility of a polyadenylation signal sequence. The effector stem comprises a polyadenylation signal. In one embodiment, the effector stem-loop comprises a polyadenylation signal sequence and sequence complimentary a polyadenylation signal sequence. In some embodiments, the stem portion of the effector stem-loop comprises only a portion of the polyadenylation signal sequence. In some embodiments, part of the polyadenylation signal sequence is located in the loop portion of the effector stem-loop.

The polyadenylation signal sequence can be any sequence in the 3' UTR of the target gene that is involved in efficient polyadenylation of the mRNA transcribed from the target gene including AATAAA (or related sequences), a downstream sequence element (DSE) (e.g., T or GT-rich rich sequence), or an upstream sequence element (USE). In some embodiments, the polyadenylation signal sequence is endogenous sequence from the 3' UTR of the target gene. In other embodiments, the polyadenylation signal sequence is exogenous sequence (for example, sequence from a different gene or different organism) or synthetic polyadenylation signal sequence.

One of the stem arms of the effector stem-loop is linked to an aptamer via a stem of the aptamer *(see,* for example, Figs. 1a, 1b, and 2a). When the aptamer is not bound to its ligand, the effector stem-loop is in a context that inhibits access to a polyadenylation signal sequence, inhibiting polyadenylation and leading to degradation of the message. When the aptamer binds its ligand, the effector stem-loop is in a conformation that does not inhibit access to the polyadenylation signal sequence, allowing polyadenylation of the message and increased target gene expression.

The stem portion of the effector stem-loop should be of a sufficient length (and GC content) to promote stem-loop structure formation and thereby inhibit accessibility to the polyadenylation signal sequence when the aptamer ligand is not present in sufficient quantities. In embodiments of the invention, the stem portion of the effector stem-loop comprises stem sequence in addition to the polyadenylation signal sequence and its complementary sequence. The length and sequence of the stem portion can be modified using known techniques in order to identify stems that allow acceptable background expression of the target gene when no ligand is present and acceptable expression levels of the target gene when the ligand is present. If the stem is, for example, too long it may hide access to the polyadenylation signal sequence in the presence or absence of ligand. If the stem is too short, it may not form a stable stem-loop structure capable of sequestering the polyadenylation signal sequence, in which case polyadenylation of the message (leading to expression of the target gene) will occur in the presence or absence of ligand. In one embodiment, the total length of the effector stem (i.e., the stem-forming portion of the effector stem-loop) is 4 to 24 base pairs, 5 to 20 base pairs, 9 to 14 base pairs, 9 base pairs, 10 base pairs, 11 base pairs or 12 base pairs. In addition to the length of the stem, the GC base pair content of the stem can be altered to modify the stability of the stem. In some embodiments, the effector region stem contains one or more mismatched nucleotides that do not base pair with the complementary portion of the effector region stem.

### Alternatively shared stem arm

The effector stem-loop and the aptamer are linked via an alternatively shared stem arm that comprises sequence that is complimentary to both the non-shared arm of the effector stem-loop and the non-shared arm of the aptamer stem. Due to the sequence that is complimentary to both the aptamer and effector stems on the unshared arm, the shared stem arm can alternatively form a stem with either the aptamer stem or the effector stem (but not both simultaneously) depending on the presence of an aptamer ligand. In embodiments, the portion of the alternatively shared stem arm that is complementary to sequence in the aptamer stem and to sequence in the effector stem loop (i.e., alternatively shared sequence) is 4 to 8 nucleotides, 5 to 7 nucleotides, 5 nucleotides, or 6 nucleotides. In some embodiments, the alternatively shared stem arm comprises additional sequence that is complimentary to only one of the unshared effector stem or the aptamer stem. In some embodiments, in addition to the alternatively shared sequence, the alternatively shared stem arm comprises (a) sequence that is complementary to the unshared effector stem arm, but not complementary to the unshared aptamer stem arm; (b) sequence that is complementary to the unshared aptamer stem arm, but not complementary to the unshared effector stem arm; or (c) both.

### Aptamer/Ligand

The sensor region comprises an aptamer. The term "aptamer" as used herein refers to an RNA polynucleotide that specifically binds to a ligand. The aptamer is linked to the effector stem-loop via an aptamer stem. The aptamer stem may, or may not, comprise sequence that is typically part of the aptamer (e.g., wild-type aptamer stem sequence). As such, reference to the aptamer stem does not imply that the aptamer stem comprises any particular sequence. Thus, the aptamer stem may comprise sequence from the aptamer and/or additional sequence capable of forming a stem upon ligand/aptamer binding.

As with the stem of the effector stem-loop discussed above, the aptamer stem loop should be a sufficient length (and GC content) such that the aptamer forms the aptamer stem in the presence of aptamer ligand and the effector stem-loop forms a stem when ligand is not present. The length and sequence of the aptamer stem can be modified using known techniques in order to identify stems that allow acceptable background expression of the target gene when no ligand is present and acceptable expression levels of the target gene when the ligand is present. In embodiments, the aptamer stem is 6 to 12 base pairs, 7 to 10 base pairs, 8 base pairs, or 9 base pairs.

The term "ligand" refers to a molecule that is specifically bound by an aptamer. In one embodiment, the ligand is a low molecular weight (less than about 1,000 Daltons) molecule including, for example, lipids, monosaccharides, second messengers, cofactors, metal ions, other natural products and metabolites, nucleic acids, as well as most therapeutic drugs. In one embodiment, the ligand is a polynucleotide with two or more nucleotide bases.

In one embodiment, the ligand is selected from the group consisting of 8-azaguanine, adenosine 5'-monophosphate monohydrate, amphotericin B, avermectin B 1, azathioprine, chlormadinone acetate, mercaptopurine, moricizine hydrochloride, N6-methyladenosine, nadide, progesterone, promazine hydrochloride, pyrvinium pamoate, sulfaguanidine, testosterone propionate, thioguanosine, tyloxapol and vorinostat.

Aptamer ligands can also be cell endogenous components that increase significantly under specific physiological/pathological conditions, such as oncogenic transformation - these may include second messenger molecules such as GTP or GDP, calcium; fatty acids, or fatty acids that are incorrectly metabolized such as 13-HODE in breast cancer (Flaherty, JT et al., Plos One, Vol. 8, e63076, 2013); amino acids or amino acid metabolites; metabolites in the glycolysis pathway that usually have higher levels in cancer cells or in normal cells in metabolic diseases; and cancer-associated molecules such as Ras or mutant Ras protein, mutant EGFR in lung cancer, indoleamine-2,3-dioxygenase (IDO) in many types of cancers. Endogenous ligands include progesterone metabolites in breast cancer as disclosed by JP Wiebe (Endocrine-Related Cancer (2006) 13:717-738). Endogenous ligands also include metabolites with increased levels resulting from mutations in key metabolic enzymes in kidney cancer such as lactate, glutathione, kynurenine as disclosed by Minton, DR and Nanus, DM (Nature Reviews, Urology, Vol. 12, 2005).

Aptamers have binding regions that are capable of forming complexes with an intended target molecule (i.e., the ligand). The specificity of the binding can be defined in terms of the comparative dissociation constants (Kd) of the aptamer for its ligand as compared to the dissociation constant of the aptamer for unrelated molecules. Thus, the ligand is a molecule that binds to the aptamer with greater affinity than to unrelated material. Typically, the Kd for the aptamer with respect to its ligand will be at least about 10-fold less than the Kd for the aptamer with unrelated molecules. In other embodiments, the Kd will be at least about 20-fold less, at least about 50-fold less, at least about 100-fold less, and at least \about 200-fold less. An aptamer will typically be between about 15 and about 200 nucleotides in length. More commonly, an aptamer will be between about 30 and about 100 nucleotides in length.

The aptamers that can be incorporated as part of the riboswitch can be a naturally occurring aptamer, or modifications thereof, or aptamers that are designed de novo and/or screened through systemic evolution of ligands by exponential enrichment (SELEX) or other screening methods. Examples of aptamers that bind small molecule ligands include, but are not limited to theophylline, dopamine, sulforhodamine B, cellobiose, kanamycin A, lividomycin, tobramycin, neomycin B, viomycin, chloramphenicol, streptomycin, cytokines, cell surface molecules, and metabolites. For a review of aptamers that recognize small molecules, *see, e.g.,* Famulok, Science 9:324-9 (1999) and McKeague, M. & DeRosa, M.C. J. Nuc. Aci. 2012. In another embodiment, the aptamer is a complementary polynucleotide.

### Methods for identifying aptamer/ligand

In one embodiment, the aptamer is designed to bind a particular small molecule ligand. Methods for designing and selecting aptamers that bind particular ligands are disclosed in WO/2018/025085. Other methods for screening aptamers include, for example, SELEX. Methods for designing aptamers that selectively bind a small molecule using SELEX are disclosed in, e.g., U.S. Patent Nos. 5,475,096, 5,270,163, and Abdullah Ozer, et al. Nuc. Aci. 2014. Modifications of the SELEX process are described in U.S. Patent Nos. 5,580,737 and 5,567,588.

Selection techniques for identifying aptamers generally involve preparing a large pool of DNA or RNA molecules of the desired length that contain a region that is randomized or mutagenized. For example, an oligonucleotide pool for aptamer selection might contain a region of 20-100 randomized nucleotides flanked by regions of defined sequence that are about 15-25 nucleotides long and useful for the binding of PCR primers. The oligonucleotide pool is amplified using standard PCR techniques, or other means that allow amplification of selected nucleic acid sequences. The DNA pool may be transcribed *in vitro* to produce a pool of RNA transcripts when an RNA aptamer is desired. The pool of RNA or DNA oligonucleotides is then subjected to a selection based on their ability to bind specifically to the desired ligand. Selection techniques include, for example, affinity chromatography, although any protocol which will allow selection of nucleic acids based on their ability to bind specifically to another molecule may be used. Selection techniques for identifying aptamers that bind small molecules and function within a cell may involve cell based screening methods. In the case of affinity chromatography, the oligonucleotides are contacted with the target ligand that has been immobilized on a substrate in a column or on magnetic beads. The oligonucleotide is preferably selected for ligand binding in the presence of salt concentrations, temperatures, and other conditions which mimic normal physiological conditions. Oligonucleotides in the pool that bind to the ligand are retained on the column or bead, and nonbinding sequences are washed away. The oligonucleotides that bind the ligand are then amplified (after reverse transcription if RNA transcripts were utilized) by PCR (usually after elution). The selection process is repeated on the selected sequences for a total of about three to ten iterative rounds of the selection procedure. The resulting oligonucleotides are then amplified, cloned, and sequenced using standard procedures to identify the sequences of the oligonucleotides that are capable of binding the target ligand. Once an aptamer sequence has been identified, the aptamer may be further optimized by performing additional rounds of selection starting from a pool of oligonucleotides comprising a mutagenized aptamer sequence.

In vivo aptamer screening may be used following one or more rounds of in vitro selection (e.g., SELEX). For example, Konig, J. et al. (RNA. 2007, 13(4):614-622) describe combining SELEX and a yeast three-hybrid system for in vivo selection of aptamer.

### Target genes

The gene regulation cassette of the present invention is a platform that can be used to regulate the expression of any target gene that can be expressed in a target cell, tissue or organism with a mRNA that is polyadenylated. The term "target gene" refers to a polynucleotide that is introduced into a cell and is capable of being transcribed into RNA and translated and/or expressed under appropriate conditions. Alternatively, the target gene is endogenous to the target cell, and the gene regulation cassette of the present invention is positioned into the 3' UTR of the target gene. An example of a target gene is a polynucleotide encoding a therapeutic polypeptide. In one embodiment, the target gene is exogenous to the cell in which the recombinant DNA construct is to be transcribed. In another embodiment, the target gene is endogenous to the cell in which the recombinant DNA construct is to be transcribed.

The target gene according to the present invention may be a gene encoding a protein. The target gene may be, for example, a gene encoding a structural protein, an enzyme, a cell signaling protein, a mitochondrial protein, a zinc finger protein, a hormone, a transport protein, a growth factor, a cytokine, an intracellular protein, an extracellular protein, a transmembrane protein, a cytoplasmic protein, a nuclear protein, a receptor molecule, an RNA binding protein, a DNA binding protein, a transcription factor, translational machinery, a channel protein, a motor protein, a cell adhesion molecule, a mitochondrial protein, a metabolic enzyme, a kinase, a phosphatase, exchange factors, a chaperone protein, and modulators of any of these. In embodiments, the target gene encodes erythropoietin (Epo), human growth hormone (hGH), transcription activator-like effector nucleases (TALEN), human insulin, CRISPR associated protein 9 (cas9), or an immunoglobulin (or portion thereof), including, e.g., a therapeutic antibody.

### Expression Constructs

The present invention contemplates the use of a recombinant vector for introduction into target cells, *in vitro* or *ex vivo,* of a polynucleotide encoding a target gene and containing a gene regulation cassette described herein. In many embodiments, the recombinant DNA construct of this invention includes additional DNA elements including DNA segments that provide for the replication of the DNA in a host cell and expression of the target gene in that cell at appropriate levels. The ordinarily skilled artisan appreciates that expression control sequences (promoters, enhancers, and the like) are selected based on their ability to promote expression of the target gene in the target cell. "Vector" means a recombinant plasmid, yeast artificial chromosome (YAC), mini chromosome, DNA mini-circle or virus (including virus derived sequences) that comprises a polynucleotide to be delivered into a host cell, either in vitro or in vivo. In one embodiment, the recombinant vector is a viral vector or a combination of multiple viral vectors.

Viral vectors for the aptamer-mediated expression of a target gene in a target cell, tissue, or organism are known in the art and include adenoviral (AV) vectors, adeno-associated virus (AAV) vectors, retroviral and lentiviral vectors, and Herpes simplex type 1 (HSV1) vectors.

Adenoviral vectors include, for example, those based on human adenovirus type 2 and human adenovirus type 5 that have been made replication defective through deletions in the E1 and E3 regions. The transcriptional cassette can be inserted into the E1 region, yielding a recombinant E1/E3-deleted AV vector. Adenoviral vectors also include helper-dependent high-capacity adenoviral vectors (also known as high-capacity, "gutless" or "gutted" vectors), which do not contain viral coding sequences. These vectors, contain the cis-acting elements needed for viral DNA replication and packaging, mainly the inverted terminal repeat sequences (ITR) and the packaging signal (Ψ). These helper-dependent AV vector genomes have the potential to carry from a few hundred base pairs up to approximately 36 kb of foreign DNA.

Recombinant adeno-associated virus "rAAV" vectors include any vector derived from any adeno-associated virus serotype, including, without limitation, AAV-1, AAV-2, AAV-3, AAV-4, AAV-5, AAV-7 and AAV-8, AAV-9, AAV-10, and the like. rAAV vectors can have one or more of the AAV wild-type genes deleted in whole or in part, preferably the Rep and/or Cap genes, but retain functional flanking ITR sequences. Functional ITR sequences are retained for the rescue, replication, packaging and potential chromosomal integration of the AAV genome. The ITRs need not be the wild-type nucleotide sequences, and may be altered (e.g., by the insertion, deletion or substitution of nucleotides) so long as the sequences provide for functional rescue, replication and packaging.

Alternatively, other systems such as lentiviral vectors can be used in embodiments of the invention. Lentiviral-based systems can transduce non-dividing as well as dividing cells making them useful for applications targeting, for examples, the non-dividing cells of the CNS. Lentiviral vectors are derived from the human immunodeficiency virus and, like that virus, integrate into the host genome providing the potential for long-term gene expression.

Polynucleotides, including plasmids, YACs, minichromosomes and minicircles, carrying the target gene containing the gene regulation cassette can also be introduced into a cell or organism by nonviral vector systems using, for example, cationic lipids, polymers, or both as carriers. Conjugated poly-L-lysine (PLL) polymer and polyethylenimine (PEI) polymer systems can also be used to deliver the vector to cells. Other methods for delivering the vector to cells includes hydrodynamic injection and electroporation and use of ultrasound, both for cell culture and for organisms. For a review of viral and non-viral delivery systems for gene delivery see Nayerossadat, N. et al. (Adv Biomed Res. 2012; 1:27).

### Methods of Modulating Expression of a Target Gene

In one aspect, this invention provides a method of modulating expression of a target gene (e.g., a therapeutic gene), by (a) inserting one or more gene regulation polynucleotide cassettes of the present invention into the 3' UTR of a target gene; (b) introducing the target gene comprising the gene regulation cassette into a cell *in vitro* or *ex vivo;* and (c) exposing the cell to a ligand that binds the aptamer. In one embodiment, the ligand is a small molecule. In aspects, expression of the target gene in target cells confers a desired property to a cell into which it was introduced, or otherwise leads to a desired therapeutic outcome. The target cells are Eukaryotic cells, for example mammalian cells. In embodiments, target cells are human cells from a target tissue, including, for example, adipose, central nervous system (CNS), muscle, cardiac, ocular, hepatic, and the like.

In one embodiment, one or more gene regulation cassettes are inserted into the 3' untranslated region of the target gene. In one embodiment, a single gene regulation cassette is inserted into the 3' UTR of a target gene. In one embodiment, two riboswitches are inserted into the 3' untranslated region of the target gene, wherein the effector stem loop of the first riboswitch comprises all or part of the polyadenylation signal AATAAA (or ATTAA) and the effector stem loop of the second riboswitch comprises all or part of the downstream element (DSE).

In one embodiment, when multiple gene regulation cassettes are inserted into a target gene, they each can contain the same aptamer such that a single ligand can be used to modulate expression of the target gene. In other embodiments, when multiple gene regulation cassettes are inserted into a target gene, each can contain a different aptamer so that exposure to multiple different small molecule ligands modulates target gene expression.

The polynucleotide cassette of the present invention can be used in combination with other mechanisms for the regulation of expression of the target gene. In one embodiment, a polynucleotide cassette of the present invention is used in combination with a gene regulation cassette that modulates target gene expression by aptamer-mediated regulation of alternative splicing as described in WO 2016/126747 The present invention can also be combined with the polynucleotide constructs and methods described in PCT/US2017/016303 and PCT/US 1207/016279.

### Medical uses and Pharmaceutical Compositions

One aspect of the invention provides the polynucleotide cassette or vector described herein for use in a method of regulating the level of a therapeutic protein delivered by gene therapy. In this embodiment, the "target gene" may encode the therapeutic protein. The "target gene" may encode a protein that is endogenous or exogenous to the cell.

The therapeutic gene sequence containing the regulatory cassette with aptamer-driven riboswitch is delivered to target cells in vitro or ex vivo, e.g., by a vector. The cell specificity of the "target gene" may be controlled by promoter or other elements within the vector. Delivery of the vector construct containing the target gene and the polynucleotide cassette, and the transfection of the target tissues resulting in stable transfection of the regulated target gene, is often the first steps in producing the therapeutic protein.

However, due to the presence of the regulatory cassette within the target gene sequence, the target gene is not expressed at significant levels (or is expressed at lower levels), i.e., it is in the "off state" in the absence of the specific ligand that binds to the aptamer contained within in the regulatory cassette riboswitch. Only when the aptamer specific ligand is administered (or otherwise present in sufficient quantities) is the target gene expression activated or increased.

The delivery of the vector construct containing the target gene with the polynucleotide cassette and the delivery of the activating ligand generally are separated in time. The delivery of the activating ligand will control when the target gene is expressed, as well as the level of protein expression. The ligand may be delivered by a number of routes including, but not limited to, oral, intramuscular (IM), intravenous (IV), intraocular, or topically.

The timing of delivery of the ligand will depend on the requirement for activation of the target gene. For example, if the therapeutic protein encoded by the target gene is required constantly, an oral small molecule ligand may be delivered daily, or multiple times a day, to ensure continual activation of the target gene, and thus continual expression of the therapeutic protein. If the target gene has a long acting effect, the inducing ligand may be dosed less frequently.

This invention allows the expression of the therapeutic transgene to be controlled temporally, in a manner determined by the temporal dosing of the ligand specific to the aptamer within the riboswitch of the regulatory polynucleotide cassette. The increased expression of the therapeutic transgene only on ligand administration, increases the safety of a gene therapy treatment by allowing the target gene to be off in the absence of the ligand.

Different aptamers can be used to allow different ligands to activate target genes. In certain embodiments of the invention, each therapeutic gene containing a regulatory cassette will have a specific aptamer within the cassette that will be activated by a specific small molecule. This means that each therapeutic gene can be activated only by the ligand specific to the aptamer housed within it. In these embodiments, each ligand will only activate one therapeutic gene. This allows for the possibility that several different "target genes" may be delivered to one individual and each will be activated on delivery of the specific ligand for the aptamer contained within the regulatory cassette housed in each target gene.

This invention allows any therapeutic protein whose gene can be delivered to the body (such as erythropoietin (EPO) or a therapeutic antibody) to be produced by the body when the activating ligand is delivered. This method of therapeutic protein delivery may replace the manufacture of such therapeutic proteins outside of the body which are then injected or infused, e.g., antibodies used in cancer or to block inflammatory or autoimmune disease. The body containing the regulated target gene becomes the biologics manufacturing factory, which is switched on when the gene-specific ligand is administered.

Dosing levels and timing of dosing of a therapeutic protein may be important to therapeutic effect. For example, in the delivery of AVASTIN (anti-VEGF antibody) for cancer. The present invention increases the ease of dosing in response to monitoring for therapeutic protein levels and effects.

**In** one embodiment, the target gene may encode a nuclease that can target and edit a particular DNA sequence. Such nucleases include Cas9, zinc finger containing nucleases, or TALENs. In the case of these nucleases, the nuclease protein may be required for only a short period of time that is sufficient to edit the target endogenous genes. However, if an unregulated nuclease gene is delivered to the body, this protein may be present for the rest of the life of the cell. In the case of nucleases, there is an increasing risk of off-target editing the longer the nuclease is present. Regulation of expression of such proteins has a significant safety advantage. In this case, vector containing the nuclease target gene containing a regulatory cassette could be delivered to the appropriate cells in the body. The target gene is in the "off" state in the absence of the cassette-specific ligand, so no nuclease is produced. Only when the activating ligand is administered, is the nuclease produced. When sufficient time has elapsed allowing sufficient editing to occur, the ligand will be withdrawn and not administered again. Thus, the nuclease gene is thereafter in the "off" state and no further nuclease is produced and editing stops. This approach may be used to correct genetic conditions, including a number of inherited retinopathies such as LCA10 caused by mutations in CEP290 and Stargardt's Disease caused by mutations in ABCA4.

Administration of a regulated target gene encoding a therapeutic protein which is activated only on specific ligand administration may be used to regulate therapeutic genes to treat many different types of diseases, e.g., cancer with therapeutic antibodies, immune disorders with immune modulatory proteins or antibodies, metabolic diseases, rare diseases such as PNH with anti-C5 antibodies or antibody fragments as the regulated gene, or ocular angiogenesis with therapeutic antibodies, and dry AMD with immune modulatory proteins.

A wide variety of specific target genes, allowing for the treatment of a wide variety of specific diseases and conditions, are suitable for use in the present invention. For example, insulin or an insulin analog (preferably human insulin or an analog of human insulin) may be used as the target gene to treat type I diabetes, type II diabetes, or metabolic syndrome; human growth hormone may be used as the target gene to treat children with growth disorders or growth hormone-deficient adults; erythropoietin (preferably human erythropoietin) may be used as the target gene to treat anemia due to chronic kidney disease, anemia due to myelodysplasia, or anemia due to cancer chemotherapy.

The present invention may be especially suitable for treating diseases caused by single gene defects such as cystic fibrosis, hemophilia, muscular dystrophy, thalassemia, or sickle cell anemia. Thus, human β-, γ-, δ-, or ζ-globin may be used as the target gene to treat β-thalassemia or sickle cell anemia; human Factor VIII or Factor IX may be used as the target gene to treat hemophilia A or hemophilia B.

The ligands used in the present invention are generally combined with one or more pharmaceutically acceptable carriers to form pharmaceutical compositions suitable for administration to a patient. Pharmaceutically acceptable carriers include solvents, binders, diluents, disintegrants, lubricants, dispersion media, coatings, antibacterial and antifungal agents, isotonic and absorption delaying agents, and the like, generally used in the pharmaceutical arts. Pharmaceutical compositions may be in the form of tablets, pills, capsules, troches, and the like, and are formulated to be compatible with their intended route of administration. Examples of routes of administration include parenteral, e.g., intravenous, intradermal, intranasal, subcutaneous, oral, inhalation, transdermal (topical), transmucosal, and rectal.

The pharmaceutical compositions comprising ligands are administered to a patient in a dosing schedule such that an amount of ligand sufficient to desirably regulate the target gene is delivered to the patient. When the ligand is a small molecule and the dosage form is a tablet, capsule, or the like, preferably the pharmaceutical composition comprises from 0.1 mg to 10 g of ligand; from 0.5 mg to 5 g of ligand; from 1 mg to 1 g of ligand; from 2 mg to 750 mg of ligand; from 5 mg to 500 mg of ligand; or from 10 mg to 250 mg of ligand.

The pharmaceutical compositions may be dosed once per day or multiple times per day (e.g., 2, 3, 4, 5, or more times per day). Alternatively, pharmaceutical compositions may be dosed less often than once per day, e.g., once every 2, 3, 4, 5, 6, 7, 8, 9, 10, 11, 12, 13, or 14 days, or once a month or once every few months. In some embodiments of the invention, the pharmaceutical compositions may be administered to a patient only a small number of times, e.g., once, twice, three times, etc.

The present invention provides a use of the polynucleotide cassette or vector described herein for use in a method of treating a patient in need of regulated expression of a therapeutic protein encoded by a target gene. The method comprises administering to the patient a pharmaceutical composition comprising a ligand for an aptamer, where the patient previously had been administered a recombinant vector comprising the target gene, where the target gene contains a gene regulation cassette of the present invention that provides the ability to regulate expression of the target gene by the ligand of the aptamer through accessibility of one or more polyadenylation signals. Administration of the ligand increases expression of the therapeutic protein.

### Articles of Manufacture and Kits

Also described herein are kits or articles of manufacture for use in the methods described herein. The kits may comprise the compositions described herein (e.g., for compositions for delivery of a vector comprising the target gene containing the gene regulation cassette) in suitable packaging. Suitable packaging for compositions (such as ocular compositions for injection) described herein are known in the art, and include, for example, vials (such as sealed vials), vessels, ampules, bottles, jars, flexible packaging (e.g., sealed Mylar or plastic bags), and the like. These articles of manufacture may further be sterilized and/or sealed.

The present disclosure also describes kits comprising compositions described herein and may further comprise instruction(s) on methods of using the composition, such as uses described herein. The kits described herein may further include other materials desirable from a commercial and user standpoint, including other buffers, diluents, filters, needles, syringes, and package inserts with instructions for performing the administration, including e.g., any methods described herein. For example the kit may comprise rAAV for expression of the target gene comprising the gene regulation cassette of the present invention, a pharmaceutically acceptable carrier suitable for injection, and one or more of: a buffer, a diluent, a filter, a needle, a syringe, and a package insert with instructions for performing the injections. The kit may be suitable for intraocular injection, intramuscular injection, intravenous injection and the like.

"Homology" and "homologous" as used herein refer to the percent of identity between two polynucleotide sequences or between two polypeptide sequences. The correspondence between one sequence to another can be determined by techniques known in the art. For example, homology can be determined by a direct comparison of two polypeptide molecules by aligning the sequence information and using readily available computer programs. Two polynucleotide or two polypeptide sequences are "substantially homologous" to each other when, after optimally aligned with appropriate insertions or deletions, at least about 80%, at least about 85%, at least about 90%, and at least about 95% of the nucleotides or amino acids, respectively, match over a defined length of the molecules, as determined using the methods above.

"Percent sequence identity" with respect to a reference polypeptide or nucleic acid sequence is defined as the percentage of amino acid residues or nucleotides in a candidate sequence that are identical with the amino acid residues or nucleotides in the reference polypeptide or nucleic acid sequence, after aligning the sequences and introducing gaps, if necessary, to achieve the maximum percent sequence identity. Alignment for purposes of determining percent amino acid or nucleic acid sequence identity can be achieved in ways known to the ordinarily-skilled artisan, for example, using publicly available computer software programs including BLAST, BLAST-2, ALIGN or Megalign (DNASTAR) software.

The term "polynucleotide" or "nucleic acid" as used herein refers to a polymeric form of nucleotides of any length, either ribonucleotides or deoxyribonucleotides. Thus, this term includes, but is not limited to, single-, double- or multi- stranded DNA or RNA, genomic DNA, cDNA, DNA-RNA hybrids, or a polymer comprising purine and pyrimidine bases, or other natural, chemically or biochemically modified, non-natural, or derivatized nucleotide bases.

"Heterologous" or "exogenous" means derived from a genotypically distinct entity from that of the rest of the entity to which it is compared or into which it is introduced or incorporated. For example, a polynucleotide introduced by genetic engineering techniques into a different cell type is a heterologous polynucleotide (and, when expressed, can encode a heterologous polypeptide). Similarly, a cellular sequence (e.g., a gene or portion thereof) that is incorporated into a viral vector is a heterologous nucleotide sequence with respect to the vector.

It is to be understood and expected that variations in the principles of invention herein disclosed can be made by one skilled in the art. The following Examples further illustrate the invention, but should not be construed to limit the scope of the invention in any way.

### EXAMPLES

### Example 1. Regulation of target gene expression by aptamer-mediated modulation of the accessibility of polyA signal element AATAAA

### Experimental procedure:

Plasmid constructs: the EGFP gene in the pEGFP-C 1 vector (Clontech) was replaced with the firefly luciferase gene coding sequence to generate the pFLuc-SV40 vector that contains the SV40 early polyadenylation signal sequence. A DNA segment containing sequences for xpt-Guanine aptamer, an effector stem loop structure and SV40 early polyA sequences were synthesized (IDT). The synthesized DNA fragments were digested with HpaI and MluI restriction enzymes and cloned into pFLuc-SV40 digested with HpaI and MluI. Construct sequences were verified by DNA sequencing (Genewiz).

Transfection and aptamer ligand treatment: 3.5 x10⁴ HEK 293 cells were plated in a 96-well flat bottom plate the day before transfection. Plasmid DNA (500 ng) was added to a tube or a 96-well U-bottom plate. Separately, TransIT-293 reagent (Mirus; 1.4 µl) was added to 50 µl opti-mem I media (Life Technologies), and allowed to sit for 5 minutes at room temperature ("RT"). Then, 50 µl of this diluted transfection reagent was added to the DNA, mixed, and incubated at RT for 20 min. Finally, 7 µl of this solution was added to a well of cells in a 96-well plate. Four hours after transfection, the media was aspirated, and new media was added with (i) DMSO (0.5%) or 500 µM guanosine; or (ii) NaOH (2mM) as solvent control or 500 µM guanine. The induction fold was expressed as the quotient of luciferase activity obtained in the presence of aptamer ligand divided by the value obtained in the absence of the aptamer ligand.

Firefly luciferase assay of cultured cells: Twenty-four hours after media change, plates were removed from the incubator, and equilibrated to RT for several minutes on a lab bench, then aspirated. Glo-lysis buffer (Promega, 100 µL, RT) was added, and the plates allowed to remain at RT for at least 5 minutes. Then, the well contents were mixed by 50 µL trituration, and 20 µL of each sample was mixed with 20 µL of bright-glo reagent (Promega) that had been diluted to 10% in glo-lysis buffer. 96 wells were spaced on an opaque white 384-well plate. Following a 5-min incubation at RT, luminescence was measured using Tecan machine with 500 mSec read time. The luciferase activity was expressed as mean relative light unit (RLU)±S.D.

Transfection and measurement of GFP fluorescence: 3.5 x10^4 HEK 293 cells were plated in 96-well flat bottom plate the day before transfection. Plasmid DNA (500 ng) was added to a tube or a 96-well U-bottom plate. Separately, TransIT-293 reagent (Mirus; 1.4 µl) was added to 50 µl Opti-mem I media (Life Technologies), and allowed to sit for 5 minutes at RT. Then, 50 µl of this diluted transfection reagent was added to the DNA, mixed, and incubated at RT for 20 min. Finally, 7 µl of this solution was added to a well of cells in a 96-well plate. GFP fluorescence intensity was measured by Tecan plate reader, using Excitation wavelength at 484 nm, Emission wavelength at 510 nm and Excitation bandwidth at 5 nm. The GFP fluorescence intensity was expressed as the value generated by GFP constructs subtracted by the value generated by cells without transfection.

### Results:

The polyadenylated tail at 3' end of mRNA plays important roles in mRNA stability, nuclear export and translation efficiency. In order to regulate the process of pre-mRNA polyadenylation and thereby regulate target gene expression, strategies that modulate the accessibility of the polyadenylation sequence elements AATAAA (or its close variants) or the T or GT-rich downstream sequence element (DSE) were developed.

In one strategy (strategy 1), as illustrated in Figs. 1a and 1b, a stem-loop structure (the effector stem loop), in which the AATAAA polyadenylation signal sequence element is embedded in the stem of the stem-loop forming structure (the effector stem-loop). The 3' end of an aptamer was linked the 5' end of this stem loop forming structure, by a shared stem arm that contains sequence complimentary to a portion of the 5' aptamer stem arm and complimentary to a portion of the 3' stem arm of the effector stem-loop *(see e.g.,* Figs. 1a and 1b). In this configuration, in the absence of aptamer ligand, the stem-loop structure sequesters the AATAAA element, thereby suppressing polyadenylation and gene expression. However, in the presence of aptamer ligand, aptamer/ligand binding results in the formation of aptamer P1 stem, thereby disrupting the stem-loop structure, which leads to the release of AATAAA element from being sequestered and the subsequent gene expression. Thus, this configuration of genetic elements creates aptamer ligand-responsive on-riboswitch.

This strategy was tested using an xpt-guanine aptamer with the SV40 early polyA sequence, in which the sequence upstream of AATAAA element in the SV40 3' UTR was shown to be indispensable for polyadenylation. Using this strategy, 4 constructs, ATA_Gua_1 through 4 (SEQ ID NO: 1-4), were generated each having the same xpt-guanine aptamer sequence linked to the 5' end of the stem-loop structure and the AATAAA sequence element being embedded in the stem of the stem-loop structure. We rationalized that the length and sequence composition for the stem of stem-loop structure can affect the stability of the stem-loop structure, thus the availability of the sequestered AATAAA element for gene expression. Therefore, 9, 10, 12 and 14 base pair (bp) stems were created, respectively, in each of the constructs. The aptamer P1 stem was 8 bp. As shown in Fig. 1c, in the absence of aptamer ligand guanosine, luciferase activity was approximately similar from all the constructs, and lower than the control construct pFLuc-SV40 (data not shown), indicating the AATAAA element was being sequestered by the stem-loop structure. Upon guanosine treatment, each construct showed enhanced luciferase activity compared to the DMSO treated (control) samples, with ATA_Gua_1 generating 6.7-fold induction.

These constructs were further tested using guanine as the aptamer ligand. As shown in Fig. 1d, in the absence of guanine treatment, the basal level expression of the construct ATA_Gua_1 to 4 was reduced, with ATA_Gua_4 having the lowest basal expression (16% of the pFLuc control vector). Upon guanine treatment, the luciferase expression was enhanced compared to the samples without guanine, generating approximately 2.5-fold induction for all the constructs.

These riboswitch constructs (strategy 1) were also used to regulate GFP expression. The ATA_Gua_2 and 4 constructs, which have relatively lower basal level expression in luciferase gene (Figs. 1c, 1d), were used to generate pEGFP_ATA_Gua_2 and 4 constructs. As shown in Fig. 1e, in the absence of guanine treatment, the GFP expression is decreased when compared to pEGFP-C1 construct. Guanine treatment produced a 1.5 and 1.6-fold increases in GFP expression (for the ATA_Gua_2 and ATA_Gua_4 constructs, respectively). The control construct, however, did not have an increase in GFP expression in response to guanine treatment.

This data demonstrates that a ligand-responsive mammalian on-riboswitch is effective at regulating target gene expression by modulating the accessibility of a polyadenylation signal sequence element in a stem-loop structure via an adjacent aptamer. Fold induction can be improved by lowering the basal expression level and increasing the induced target gene expression of the construct through optimizing the length and sequence of the aptamer P1 stem and the stem in the effector stem-loop structure. In this tested configuration of genetic elements, a guanine aptamer and SV40 early polyA sequence were utilized. Similar strategies can be used to generate riboswitches using various aptamers in modulating various polyA sequences including synthetic polyA sequences.

**Example 2.** Modulation of target gene expression by aptamer/ligand mediated polyadenylation via accessibility of a downstream sequence element.

### Experimental procedures: as described in Example 1.

### Results:

Another strategy (strategy 2) was developed to modulate the polyadenylation through modulating the accessibility of T or GT-rich downstream sequence element (DSE). In this strategy, as illustrated in Fig. 2a, a stem-loop structure was created, in which the DSE sequence is embedded in the stem of the stem-loop structure. An aptamer sequence was linked to the 3' end of this stem-loop structure followed by the complementary sequence of the 3' arm of the stem-loop structure. In this configuration, in the absence of aptamer ligand, the stem-loop structure sequesters the DSE sequence, inhibiting polyadenylation and thereby suppressing gene expression. However, in the presence of aptamer ligand, aptamer/ligand binding results in the formation of the aptamer P1 stem, disrupting the effector stem-loop structure and releasing the DSE sequence from being sequestered, and allowing the gene expression. Thus, similar to the strategy demonstrated in Example 1, this configuration of genetic elements creates an aptamer ligand-responsive on-riboswitch.

This strategy was tested using xpt-guanine aptamer with the SV40 early polyA sequence, in which DSE sequence, together with AATAAA element in the SV40 3' UTR, was shown to be responsible for pre-mRNA polyadenylation. Using this strategy, 4 constructs, DSE_Gua_1 through 4 (SEQ ID NO: 5-8), were generated each having the same xpt-guanine aptamer sequence linked to the 3' end of the stem-loop structure with the DSE sequence embedded in the stem of the stem-loop structure. We rationalized that the length and sequence composition for the stem of stem-loop structure can affect the stability of the stem-loop structure, thus the availability of the sequestered DSE sequence for gene expression. Therefore, 9, 10, 12 and 14 base pair (bp) stems were created, respectively, in each of these constructs. An aptamer P1 stem of 8 bp or 9 bp was utilized in these constructs. As shown in Fig. 2b, in the absence of aptamer ligand guanosine, the luciferase activity was approximately similar from constructs DSE_Gua_1 to 3, with DSE_Gua_4 being the lowest, lower than the control construct pFLuc-SV40 (data not shown), indicating that the DSE element was sequestered by the stem-loop structure in the absence of aptamer ligand. Whereas, upon guanosine treatment, each construct showed enhanced luciferase activity when compared to the DMSO treated samples, with DSE_Gua_2, e.g., generating 3.7-fold induction.

These constructs were also tested using guanine as aptamer ligand. As shown in Fig. 2c, in the absence of guanine treatment, the luciferase activity was decreased to approximately 68% for construct DSE_Gua_1 to 3, and 80% for construct DSE_Gua_4, when compared to control construct pFLuc. In the presence of guanine, luciferase expression was increased by approximately 2.4-fold for all the constructs when compared to the samples without guanine treatment. Fold induction can be further improved by lowering the basal level expression and increasing the induced gene expression of the construct through optimizing the length and sequence of the stem in the stem-loop structure, as well as for the aptamer P1 stem. These data further demonstrate the creation of an aptamer ligand-responsive mammalian on-riboswitch that regulates gene expression through modulating the accessibility of polyadenylation sequence element via an aptamer. In this tested configuration of genetic elements, guanine aptamer and SV40 early polyA sequence was used. Similar strategies can be used to generate riboswitches containing various aptamers in modulating various polyA sequences including synthetic polyA sequences.

### Example 3. Use of guanine aptamer to modulate the accessibility of synthetic polyA sequence elements in the 3'UTR

### Experimental Procedure:

Plasmid construction: DNA fragments containing sequences for synthetic polyA (SPA) (Levitt, N. et al., Definition of an efficient synthetic poly(A) site, Genes & Development. 1989; 3:1019-1025), or SPA from pGLuc-Basic_2 (NEB) named here as mtSPA, or xpt-guanine aptamer, stem loop structure and synthetic polyA sequences, were synthesized (IDT). The synthesized DNA fragments were digested with XhoI and NheI restriction enzymes and cloned into Con8 construct digested with XhoI and XbaI to generate Con8-SPA (SEQ ID NO: 9), SPA_ATA_Gua (SEQ ID NO: 10), SPA_DSE_Gua (SEQ ID NO: 11), mtCon8-SPA (SEQ ID NO: 12) and mtSPA_ATA_Gua (SEQ ID NO: 13). Construct sequences were verified by DNA sequencing (Genewiz).

Transfection, aptamer ligand treatment and luciferase assay of cultured cells: as described in Example 1.

### Results:

Regulation of target gene expression through aptamer-mediated modulation of accessibility of SV40 polyA sequence is demonstrated in Examples 1 and 2. The aptamer-modulated polyadenylation, was also tested using a synthetic polyA sequence (SPA). Using same strategy as demonstrated in Example 1, the construct SPA_ATA_Gua was generated with the xpt-guanine aptamer sequence linked to the 5' end of the stem-loop structure and the AATAAA sequence element of synthetic polyA sequence being embedded in the stem of the stem-loop structure. A construct SPA_DSE_Gua was also generated using the same strategy as demonstrated in Example 2. As shown in Fig. 3, in the absence of guanine treatment, linking of the stem-loop structure and xpt-guanine aptamer did not result in reduced luciferase activity in the SPA_ATA_Gua construct, suggesting inefficient sequestering in the stem-loop of the AATAAA sequence element of the synthetic polyA sequence. To address this, another SPA sequence (mtSPA) was used, which has 2 nt difference, but shows similar functionality when compared to Con8-SPA. In order to strengthen the stem, 2 GC base pairs were added in the stem, together with the 7 nt of the polyA sequence and its complementary sequence, a 9bp stem was generated. With this mtSPA_ATA_Gua construct, the basal level expression of luciferase activity was decreased when compared to mtCon8-SPA in the absence of guanine treatment. Whereas, in the presence of guanine treatment, the luciferase activity was slightly upregulated to 1.7 fold when comparing to samples without guanine treatment. With construct SPA_DSE_Gua, xpt-guanine aptamer sequence was linked to the 3' end of the stem-loop structure with the DSE sequence being embedded in the stem of the stem-loop structure. As shown in Figure 3, guanine treatment upregulated the luciferase activity of SPA_DSE_Gua construct to 1.9 fold when compared to the samples without guanine treatment. These results demonstrate the regulatability of target gene expression by modulating the accessibility of polyA sequence elements.

### Example 4. Modulating loop sequence of the stem-loop structure to enhance regulatability of the polyA-based riboswitch

Experimental Procedure: as described in Example 1.

### Results:

With all the constructs that have aptamers and stem loop structures linked to the polyA sequences in Examples 1 and 2, the basal level expression of luciferase is reduced to 20 to 45% of the control vector. Though the induced level of target gene expression reaches maximally 80% of the control construct, the basal level expression causes the fold induction to be lower. To reduce basal expression, the stem-loop structures can be strengthened or stablized to efficiently sequester or block the AATAAA or DSE sequence elements in the absence of aptamer/ligand binding. To strengthen the stability of the stem-loop structure, the effect of the sequence composition of the loop of the stem-loop structure on the basal level expression of luciferase gene was tested. The GAAA loop sequence in ATA_Gua_1 was replaced with a more stable loop sequence TTCG (V.P. Antao, S. Y. Lai and I. Tinoco, A thermodynamic study of unusually stable RNA and DNA hairpins. Nucleic Acids Research. 1991; 19(21):5901-5905), generating construct Stbl_ATA_Gua_1 (SEQ ID NO: 14). As shown in Fig. 4, comparing to ATA_Gua_1, Stbl_ATA_Gua_1 expressed lower luciferase activity in the absence of guanine treatment, suggesting an improved sequestering of AATAAA signal. Whereas, in the presence of guanine treatment, the luciferase activity induced for Stbl_ATA_Gua_1 was similar to ATA_Gua_1, thus generating a higher induction fold. In contrast, the construct containing GAAT loop, gaat_ATA_Gua_1 (SEQ ID NO: 15), expressed higher basal level luciferase than ATA_Gua_1 or stbl_ATA_Gua_1, generating a slightly lower fold induction than these two constructs. These results indicate that more stringent target gene regulation can be achieved through modifying the stem-loop structure.

### Example 5. Simultaneously modulating the accessibility of both AATAAA and DSE of polyA sequence in 3' UTR.

### Experimental Procedure:

Sequence containing ATA_Gua_1 and DSE_Gua_1 switches was synthesized and cloned into HpaI and MluI digested pFLuc vector to generate the ATA_DSE_Gua construct (SEQ ID NO: 16).

Transfection and luciferase assay of cultured cells: as described in Example 1.

### Results:

A third strategy for regulating target gene expression (strategy 3), as illustrated in Fig. 5a, was developed by combining strategy 1 and strategy 2 thereby modulating the accessibility of both AATAAA and DSE sequences simultaneously. In this configuration, in the absence of aptamer ligand, both AATAAA and DSE sequences are sequestered by the stem-loop structures, therefore gene expression is suppressed. Each stem-loop structure is connected to aptamer sequence that can respond to the same or different ligand molecules. Blocking both essential polyA sequence elements simultaneously can lower the basal level of gene expression in the absence of the ligand(s). In the presence of aptamer ligand, aptamer/ligand binding facilitates the formation of aptamer P1 stem, disrupting the stem-loop structures and releasing both AATAAA and DSE sequences from being sequestered. In this configuration, gene can express efficiently only when ligands for both aptamers are present, potentially allowing a more stringent regulation of target gene expression.

Indeed, as shown in Fig. 5b, in the absence of guanine treatment, sequestering both AATAAA and DSE sequences through the stem-loop structures (ATA_DSE_Gua) further reduced luciferase expression to 20% of the control construct pFLuc, while ATA_Gua_1 and DSE_Gua_1 were reduced by 45% and 35%, respectively. In the presence of guanine treatment, luciferase activity was restored by approximately 2.1 fold in ATA_DSE_Gua sample when compared to the untreated samples, indicating a more stringent gene regulation.

### Example 6. Combined use of polyA-based riboswitch and a second riboswitch to enhance the target gene regulatability

### Experimental Procedure:

The G15 riboswitch cassette *(see* examples 5 and 8 and SEQ ID NO.: 46 of WO 2016/126747) or a control cassette without aptamer was cloned into pFLuc to generate pFLuc-G15 or pFLuc-Con1, using Golden Gate cloning strategy. To generate construct pFLuc-G15_ATA_Gua, the fragment containing the guanine aptamer, stem loop structure and SV40 polyA sequences was released from ATA_Gua_1 construct by HpaI and MluI digestion and cloned into pFLuc-G15 construct digested with the same restriction enzymes.

Transfection and luciferase assay of cultured cells: as described in Example 1.

### Results:

The expression constructs containing the ATA_Gua and DSE_Gua riboswitches have a degree of basal expression of the target gene. A combined use of these switches with a second switch in tandem can tighten the basal expression and therefore enhance gene regulatability.

To demonstrate this, the G15 riboswitch was combined with the polyA-based riboswitch. G15 riboswitch is based on xpt-guanine aptamer-modulated alternative splicing mechanism and has some basal level expression, that reduces the fold induction in response to aptamer ligand guanine treatment. To reduce the basal level expression from pFLuc-G15 construct, the polyA sequence was replaced with ATA_Gua_1, generating pFLuc-G15-ATA_Gua construct (Fig. 6a). As shown in Fig. 6b, in the absence of aptamer ligand (guanine), the basal level expression of luciferase of pFLuc-G15 is substantially reduced when compared to the pFLuc-Con1 control construct. pFLuc-G15-ATA_Gua, the construct with dual switches, has further reduced basal level expression when compared to pFLuc-G15. In the presence of guanine treatment, the induced level of luciferase activity is slightly lower than pFLuc-G15, but the same as the induced luciferase activity from ATA_Gua_1 construct, generating a higher fold induction than both the ATA_Gua_1 and pFLuc-G15 constructs (Fig. 6b).

These results demonstrate that a more stringent switch can be generated by combining in tandem two switches that have higher basal level expression. The aptamers in this dual switch can be the same or different aptamers that bind to the same or different ligands as demonstrated here, or aptamers responding to different ligands.

### Example 7. Use of adenine aptamer to modulate the accessibility of polyA sequence element AATAAA in 3'UTR

### Experimental procedure:

Plasmid construction: a DNA fragment containing sequences for the adenine aptamer ydhl-A (M. Mandal and R.R. Breaker, Adenine riboswitches and gene activation by disruption of a transcription terminator. Nature Structural & Molecular Biology. 2004; 11: 29-35), stem loop structure and SV40 early polyA sequences were synthesized (IDT). The synthesized DNA fragments were digested with HpaI and MluI restriction enzymes and cloned into pFLuc-SV40 digested with HpaI and MluI. Construct sequences were verified by DNA sequencing (Genewiz).

Transfection and Luciferase assay of cultured cells: as described in Example 1.

Four hours after transfection, the media was aspirated, and new media with NaOH (1 mM) or 1 mM adenine (Calbiochem) was added. The induction fold was expressed as the quotient of luciferase activity obtained in the presence of aptamer ligand divided by the value obtained in the absence of the aptamer ligand.

### Results:

Use of additional aptamer/ligand pairs to control target gene expression was studied. With the same strategy as demonstrated in Example 1, a stem-loop (SL) structure (the stable TTCG loop) was inserted, in which the AATAAA sequence element is embedded in the 9bp stem of the SL structure. To the 5' end of this SL structure, the complementary sequence of the 5' arm of the SL structure followed by adenine aptamer ydhl-A sequence was inserted in the 3' UTR, generating construct ATA_Ydhl (SEQ ID NO: 17). In this construct, the length of ydhl aptamer P1 stem is 10 bp. As shown in Fig. 7, in the absence of adenine treatment, the ATA_Ydhl construct expressed reduced level of luciferase, approximately 52% of the control pFLuc construct, presumably through the blockade of the accessibility of the AATAAA sequence element. In the presence of adenine, the luciferase expression was enhanced when compared to the samples without adenine treatment, to approximately 82% of the control pFLuc construct in the presence of adenine treatment. Adenine treatment increased luciferase activity by 2.5-fold in the control construct through an aptamer-unrelated mechanism. However, the ATA_ydhl construct generated 4.0-fold increase in luciferase expression, indicating an adenine/aptamer specific effect.

## Claims

1. A polynucleotide cassette for the regulation of the expression of a target gene comprising a riboswitch wherein the riboswitch comprises an effector stem loop and an aptamer, wherein the effector stem comprises a polyadenylation signal, and wherein the aptamer and effector stem loop are linked by an alternatively shared stem arm comprising sequence that is complementary to the unshared arm of the aptamer stem and to the unshared arm of the effector stem loop.

2. The polynucleotide cassette of claim 1, wherein
(a) the aptamer binds a small molecule ligand;
(b) the portion of the alternatively shared stem arm that is complementary to sequence in the aptamer stem and to sequence in the effector stem loop is 4 to 8 nucleotides, 5 to 7 nucleotides, 5 nucleotides, or 6 nucleotides;
(c) the aptamer stem is 6 to 12 base pairs, 7 to 10 base pairs, 8 base pairs, or 9 base pairs; or
(d) the stem of the effector stem loop is 4 to 24 base pairs, 5 to 20 base pairs, 9 to 14 base pairs, 9 base pairs, 10 base pairs, 11 base pairs or 12 base pairs.

3. A polynucleotide cassette comprising two riboswitches of claim 1 or claim 2, wherein the effector stem loop of the first riboswitch comprises all or part of the polyadenylation signal AATAAA or ATTAAA and the effector stem loop of the second riboswitch comprises all or part of the downstream element (DSE).

4. The polynucleotide cassette of claim 3, wherein the two riboswitches
(a) each comprise an aptamer that binds the same ligand; or
(b) comprise different aptamers that bind different ligands.

5. A method of modulating the expression of a target gene comprising
(a) inserting one or more of the polynucleotide cassettes of claim 1 or claim 2 into the 3' untranslated region of a target gene,
(b) introducing the target gene comprising the polynucleotide cassette into a cell *in vitro* or ex *vivo,* and
(c) exposing the cell to a ligand that binds the aptamer in an amount effective to increase expression of the target gene;
optionally wherein the ligand is a small molecule.

6. The polynucleotide cassette of claim 1 or claim 2 or the method of claim 5, wherein the effector stem loop is positioned 3' of the aptamer such that the alternatively shared stem arm comprises all or a portion of the 3' aptamer stem arm and all or a portion of the 5' arm of the effector stem.

7. The polynucleotide cassette or the method of claim 6, wherein the polyadenylation signal is AATAAA or ATTAAA.

8. The polynucleotide cassette of claim 1 or claim 2 or the method of claim 5, wherein the effector stem loop is positioned 5' of the aptamer such that the alternatively shared stem arm comprises all or a portion of the 5' aptamer stem arm and all or a portion of the 3' arm of the effector stem.

9. The polynucleotide cassette of claim 8, wherein the polyadenylation signal is a downstream element (DSE).

10. The method of claim 5, wherein two riboswitches are inserted into the 3' UTR of the target gene, wherein the effector stem loop of the first riboswitch comprises all or part of the polyadenylation signal AATAAA or ATTAAA and the effector stem loop of the second riboswitch comprises all or part of the downstream element (DSE).

11. The method of claim 10, wherein
(a) the two riboswitches each comprise an aptamer that binds the same ligand;
(b) the two riboswitches comprise different aptamers that bind different ligands; or
(c) the two or more polynucleotide cassettes comprise the same aptamer.

12. The method according to any of claims 5 to 11, wherein
(a) the target gene comprising the polynucleotide cassette is incorporated in a vector for the expression of the target gene; or
(b) the target gene further comprises a gene regulation cassette that modulates target gene expression by aptamer- mediated regulation of alternative splicing.

13. A vector comprising a target gene that contains a polynucleotide cassette of claims 1-9.

14. The method of claim 12 or the vector of claim 13, wherein the vector is a viral vector, optionally wherein the viral vector is selected from the group consisting of adenoviral vector, adeno-associated virus vector, and lentiviral vector.

15. The vector of claim 13, wherein the target gene further comprises a gene regulation cassette that modulates target gene expression by aptamer-mediated regulation of alternative splicing.

16. The polynucleotide cassette of any one of claims 1-4 or 6-9, or the vector of claims 13 or 15 for use in the treatment of cancer, immune disorders, metabolic diseases, rare diseases such as PNH, ocular angiogenesis, dry AMD, type I diabetes, type II diabetes, metabolic syndrome, growth disorders or growth hormone-deficient, anemia due to chronic kidney disease, anemia due to myelodysplasia, anemia due to cancer chemotherapy, cystic fibrosis, hemophilia, muscular dystrophy, thalassemia, sickle cell anemia, hemophilia A or hemophilia B.

17. The polynucleotide cassette of any of claims 1-4 or 6-9, or the vector of claims 13 or 15 for use in the modulation of the expression of the target gene.

18. The polynucleotide cassette of any of claims 1-4 or 6-9, or the vector of claims 13 or 15 for use as a medicament.

## Patentansprüche

1. Polynukleotidkassette zur Regulierung der Expression eines Zielgens, die einen Riboswitch umfasst, wobei der Riboswitch eine Effektorstammschleife und ein Aptamer umfasst, wobei der Effektorstamm ein Polyadenylierungssignal umfasst und wobei das Aptamer und die Effektorstammschleife durch einen alternativ geteilten Stammarm verbunden sind, der eine Sequenz umfasst, die komplementär zum ungeteilten Arm des Aptamerstamms und zum ungeteilten Arm der Effektorstammschleife ist.

2. Polynukleotidkassette nach Anspruch 1, wobei
(a) das Aptamer einen kleinen Molekül-Liganden bindet;
(b) der Abschnitt des alternativ geteilten Stammarms, der komplementär zur Sequenz im Aptamerstamm und zur Sequenz in der Effektorstammschleife ist, 4 bis 8 Nukleotide, 5 bis 7 Nukleotide, 5 Nukleotide oder 6 Nukleotide beträgt;
(c) der Aptamerstamm 6 bis 12 Basenpaare, 7 bis 10 Basenpaare, 8 Basenpaare oder 9 Basenpaare beträgt; oder
(d) der Stamm der Effektorstammschleife 4 bis 24 Basenpaare, 5 bis 20 Basenpaare, 9 bis 14 Basenpaare, 9 Basenpaare, 10 Basenpaare, 11 Basenpaare oder 12 Basenpaare beträgt.

3. Polynukleotidkassette, umfassend zwei Riboswitches nach Anspruch 1 oder Anspruch 2, wobei die Effektorstammschleife des ersten Riboswitches das gesamte oder einen Teil des Polyadenylierungssignals AATAAA oder ATTAAA umfasst und die Effektorstammschleife des zweiten Riboswitches das gesamte oder einen Teil des Downstream-Elements (DSE) umfasst.

4. Polynukleotidkassette nach Anspruch 3, wobei die zwei Riboswitches
(a) jeweils ein Aptamer umfassen, das denselben Liganden bindet; oder
(b) verschiedene Aptamere umfassen, die unterschiedliche Liganden binden.

5. Verfahren zum Modulieren der Expression eines Zielgens, umfassend
(a) Einfügen einer oder mehrerer der Polynukleotidkassetten nach Anspruch 1 oder Anspruch 2 in die nicht-translatierte 3'-Region eines Zielgens,
(b) Einbringen des Zielgens, das die Polynukleotidkassette umfasst, in eine Zelle in vitro oder ex vivo, und
(c) Aussetzen der Zelle einem Liganden, der das Aptamer in einer Menge bindet, die wirksam ist, um die Expression des Zielgens zu erhöhen.
wobei der Ligand optional ein kleines Molekül ist.

6. Polynukleotidkassette nach Anspruch 1 oder Anspruch 2 oder Verfahren nach Anspruch 5, wobei die Effektorstammschleife 3' von dem Aptamer positioniert ist, sodass der alternativ geteilte Stammarm den gesamten oder einen Abschnitt des 3'-Aptamerstammarms und den gesamten oder einen Abschnitt des 5'-Arms des Effektorstamms umfasst.

7. Polynukleotidkassette oder das Verfahren nach Anspruch 6, wobei das Polyadenylierungssignal AATAAA oder ATTAAA ist.

8. Polynukleotidkassette nach Anspruch 1 oder Anspruch 2 oder Verfahren nach Anspruch 5, wobei die Effektorstammschleife 5' von dem Aptamer positioniert ist, sodass der alternativ geteilte Stammarm den gesamten oder einen Abschnitt des 5'-Aptamerstammarms und den gesamten oder einen Abschnitt des 3'-Arms des Effektorstamms umfasst.

9. Polynukleotidkassette nach Anspruch 8, wobei das Polyadenylierungssignal ein Downstream-Element (DSE) ist.

10. Verfahren nach Anspruch 5, wobei zwei Riboswitches in den 3'-UTR des Zielgens eingefügt werden, wobei die Effektorstammschleife des ersten Riboswitches das gesamte oder einen Teil des Polyadenylierungssignals AATAAA oder ATTAAA umfasst und die Effektorstammschleife des zweiten Riboswitches das gesamte oder einen Teil des Downstream-Elements (DSE) umfasst.

11. Verfahren nach Anspruch 10, wobei
(a) die zwei Riboswitches jeweils ein Aptamer umfassen, das denselben Liganden bindet;
(b) die zwei Riboswitches unterschiedliche Aptamere umfassen, die unterschiedliche Liganden binden; oder
(c) die zwei oder mehr Polynukleotidkassetten dasselbe Aptamer umfassen.

12. Verfahren nach einem der Ansprüche 5 bis 11, wobei
(a) das Zielgen, das die Polynukleotidkassette umfasst, in einen Vektor zur Expression des Zielgens eingebaut ist; oder
(b) das Zielgen weiter eine Genregulationskassette umfasst, welche die Expression des Zielgens durch Aptamer-vermittelte Regulierung des alternativen Spleißens moduliert.

13. Vektor, der ein Zielgen umfasst, das eine Polynukleotidkassette nach einem der Ansprüche 1-9 enthält.

14. Verfahren nach Anspruch 12 oder Vektor nach Anspruch 13, wobei der Vektor ein viraler Vektor ist, wobei der virale Vektor optional aus der Gruppe ausgewählt ist, die aus adenoviralem Vektor, adenoassoziiertem Virusvektor und lentiviralem Vektor besteht.

15. Vektor nach Anspruch 13, wobei das Zielgen weiter eine Genregulationskassette umfasst, welche die Expression des Zielgens durch Aptamer-vermittelte Regulierung des alternativen Spleißens moduliert.

16. Polynukleotidkassette nach einem der Ansprüche 1-4 oder 6-9 oder Vektor nach den Ansprüchen 13 oder 15 zur Verwendung bei der Behandlung von Krebs, Immunstörungen, Stoffwechselerkrankungen, seltenen Erkrankungen wie PNH, okulärer Angiogenese, trockener AMD, Typ-I-Diabetes, Typ-II-Diabetes, metabolischem Syndrom, Wachstumsstörungen oder Wachstumshormonmangel, Anämie aufgrund einer chronischen Nierenerkrankung, Anämie aufgrund einer Myelodysplasie, Anämie aufgrund einer Krebschemotherapie, Mukoviszidose, Hämophilie, Muskeldystrophie, Thalassämie, Sichelzellenanämie, Hämophilie A oder Hämophilie B.

17. Polynukleotidkassette nach einem der Ansprüche 1-4 oder 6-9 oder Vektor nach den Ansprüchen 13 oder 15 zur Verwendung bei der Modulation der Expression des Zielgens.

18. Polynukleotidkassette nach einem der Ansprüche 1-4 oder 6-9 oder Vektor nach den Ansprüchen 13 oder 15 zur Verwendung als Medikament.

## Revendications

1. Cassette polynucléotidique pour la régulation de l'expression d'un gène cible comprenant un riboswitch, dans laquelle le riboswitch comprend une tige-boucle d'effecteur et un aptamère, dans laquelle la tige d'effecteur comprend un signal de polyadénylation et dans laquelle l'aptamère et la tige-boucle d'effecteur sont liés par un bras de tige partagé alternativement comprenant une séquence qui est complémentaire du bras non partagé de la tige d'aptamère et du bras non partagé de la tige-boucle d'effecteur.

2. Cassette polynucléotidique selon la revendication 1, dans laquelle
(a) l'aptamère se lie à un ligand de petite molécule ;
(b) la partie du bras de tige partagé alternativement qui est complémentaire de la séquence dans la tige d'aptamère et de la séquence dans la boucle-tige d'effecteur est 4 à 8 nucléotides, 5 à 7 nucléotides, 5 nucléotides ou 6 nucléotides ;
(c) la tige d'aptamère est 6 à 12 paires de bases, 7 à 10 paires de bases, 8 paires de bases ou 9 paires de bases ; ou
(d) la tige de la boucle-tige d'effecteur est 4 à 24 paires de bases, 5 à 20 paires de bases, 9 à 14 paires de bases, 9 paires de bases, 10 paires de bases, 11 paires de bases ou 12 paires de bases.

3. Cassette polynucléotidique comprenant deux riboswitches selon la revendication 1 ou la revendication 2, dans laquelle la boucle-tige d'effecteur du premier riboswitch comprend tout ou partie du signal de polyadénylation AATAAA ou ATTAAA et la boucle-tige d'effecteur du second riboswitch comprend tout ou partie de l'élément en aval (DSE).

4. Cassette de polynucléotide selon la revendication 3, dans laquelle les deux riboswitches
(a) comprennent chacun un aptamère qui se lie au même ligand ; ou
(b) comprennent différents aptamères qui se lient à des ligands différents.

5. Procédé de modulation de l'expression d'un gène cible, comprenant
(a) l'insertion d'une ou de plusieurs des cassettes polynucléotidiques de la revendication 1 ou de la revendication 2 dans la région 3' non traduite d'un gène cible,
(b) l'introduction du gène cible comprenant la cassette de polynucléotide dans une cellule in vitro ou ex vivo, et
(c) l'exposition de la cellule à un ligand qui se lie à l'aptamère en une quantité efficace pour augmenter une expression du gène cible ;
facultativement dans lequel le ligand est une petite molécule.

6. Cassette polynucléotidique selon la revendication 1 ou la revendication 2 ou procédé selon la revendication 5, dans lequel la boucle-tige d'effecteur est positionnée en 3' de l'aptamère de telle sorte que le bras de tige partagé alternativement comprenne tout ou une partie du bras de tige d'aptamère en 3' et tout ou une partie du bras en 5' de tige d'effecteur.

7. Cassette polynucléotidique ou procédé selon la revendication 6, dans lequel le signal de polyadénylation est AATAAA ou ATTAAA.

8. Cassette polynucléotidique selon la revendication 1 ou la revendication 2 ou procédé selon la revendication 5, dans lequel la boucle-tige d'effecteur est positionnée en 5' de l'aptamère de telle sorte que le bras de tige partagé alternativement comprenne tout ou une partie du bras de tige d'aptamère en 5' et tout ou une partie du bras en 3' de la tige d'effecteur.

9. Cassette polynucléotidique selon la revendication 8, dans laquelle le signal de polyadénylation est un élément en aval (DSE).

10. Procédé selon la revendication 5, dans lequel deux riboswitches sont insérés dans l'UTR en 3' du gène cible, dans lequel la boucle-tige d'effecteur du premier riboswitch comprend tout ou partie du signal de polyadénylation AATAAA ou ATTAAA et la boucle-tige d'effecteur du second riboswitch comprend tout ou partie de l'élément en aval (DSE).

11. Procédé selon la revendication 10, dans lequel
(a) les deux riboswitches comprennent chacun un aptamère qui se lie au même ligand ;
(b) les deux riboswitches comprennent des aptamères différents qui se lient à des ligands différents ; ou
(c) les deux, ou plus, cassettes polynucléotidiques comprennent le même aptamère.

12. Procédé selon l'une quelconque des revendications 5 à 11, dans lequel
(a) le gène cible comprenant la cassette polynucléotidique est incorporé dans un vecteur pour l'expression du gène cible ; ou
(b) le gène cible comprend en outre une cassette de régulation de gène qui module une expression de gène cible par régulation d'épissage alternatif médiée par aptamère.

13. Vecteur comprenant un gène cible qui contient une cassette polynucléotidique selon l'une quelconque des revendications 1-9.

14. Procédé selon la revendication 12, ou vecteur selon la revendication 13, dans lequel le vecteur est un vecteur viral, facultativement dans lequel le vecteur viral est sélectionné dans le groupe se composant d'un vecteur adénoviral, d'un vecteur de virus adéno-associé et d'un vecteur lentiviral.

15. Vecteur selon la revendication 13, dans lequel le gène cible comprend en outre une cassette de régulation de gène qui module une expression de gène cible par régulation d'épissage alternatif médiée par aptamère.

16. Cassette polynucléotidique selon l'une quelconque des revendications 1-4 ou 6-9, ou vecteur selon les revendications 13 ou 15, pour une utilisation dans le traitement d'un cancer, de troubles immunitaires, de maladies métaboliques, de maladies rares telles que la PNH, de l'angiogenèse oculaire, de la DMLA sèche, du diabète de type I, du diabète de type Il, du syndrome métabolique, de troubles de la croissance ou d'un déficit en hormone de croissance, d'une anémie due à une maladie rénale chronique, d'une anémie due à une myélodysplasie, d'une anémie due à une chimiothérapie anticancéreuse, d'une fibrose kystique, de l'hémophilie, de la dystrophie musculaire, de la thalassémie, de l'anémie à hématies falciformes, de l'hémophilie A ou de l'hémophilie B.

17. Cassette polynucléotidique selon l'une quelconque des revendications 1-4 ou 6-9, ou vecteur selon les revendications 13 ou 15, pour une utilisation dans la modulation de l'expression du gène cible.

18. Cassette polynucléotidique selon l'une quelconque des revendications 1-4 ou 6-9, ou vecteur selon les revendications 13 ou 15, pour une utilisation comme médicament.
